# EUROPEAN PATENT SPECIFICATION

(11) **EP 2 627 354 B1**
(45) Date of publication and mention of the grant of the patent: **15.07.2015**
(21) Application number: 11768055.3
(22) Date of filing: 14.10.2011
(51) Int. Cl.: A61K 39/36, A61K 39/35, A61K 39/00, A61K 38/38, A61P 37/00

(54) **SUPPRESSION OF A TYPE 1 HYPERSENSITIVITY IMMUNE RESPONSE WITH AN UNRELATED ANTIGEN**
UNTERDRÜCKUNG EINER TYP-1-ÜBEREMPFINDLICHKEITSIMMUNREAKTION MIT EINEM UNZUSAMMENHÄNGENDEN ANTIGEN
SUPPRESSION D'UNE RÉPONSE IMMUNITAIRE D'HYPERSENSIBILITÉ DE TYPE 1 PAR UN ANTIGÈNE NON APPARENTÉ

(30) Priority: 11.03.2011 EP 11157868; 15.10.2010 US 393460 P; 15.10.2010 EP 10187744
(43) Date of publication of application: 21.08.2013
(73) Proprietor: ALK-Abelló A/S, 2970 Hørsholm (DK)
(72) Inventor: BRIMNES, Jens, 2730 Herlev (DK); LUND, Kaare, 2830 Virum (DK)
(74) Representative: Inspicos A/S
(86) International application number: PCT/EP2011/068028
(87) International publication number: WO 2012/049312

(56) References cited:
- WO-A1-93/16724
- WO-A1-99/56763
- WO-A1-2004/064863
- WO-A1-2004/082710
- WO-A1-2005/115449
- WO-A2-2006/050729
- US-A1- 2005 202 032
- US-A1- 2007 231 298
- MILLER A ET AL: "ANTIGEN-DRIVEN BYSTANDER SUPPRESSION AFTER ORAL ADMINISTRATION OF ANTIGENS", THE JOURNAL OF EXPERIMENTAL MEDICINE, ROCKEFELLER UNIVERSITY PRESS, US, vol. 174, no. 4, 1 October 1991 (1991-10-01), pages 791-798, XP000607046, ISSN: 0022-1007, DOI: DOI:10.1084/JEM.174.4.791 cited in the application
- DAHLMAN-HÖGLUND A ET AL: "Bystander suppression of the immune response to human serum albumin in rats fed ovalbumin", IMMUNOLOGY, BLACKWELL PUBLISHING, OXFORD, GB, vol. 86, no. 1, 1 September 1995 (1995-09-01), pages 128-133, XP002618892, ISSN: 0019-2805 cited in the application
- OWAIN R MILLINGTON ET AL: "Induction of Bystander Suppression by Feeding Antigen Occurs despite Normal Clonal Expansion of the Bystander T Cell Population", JOURNAL OF IMMUNOLOGY, AMERICAN ASSOCIATION OF IMMUNOLOGISTS, US, vol. 173, no. 10, 15 November 2004 (2004-11-15), pages 6059-6064, XP002618893, ISSN: 0022-1767 cited in the application
- OLIVEIRA C R ET AL: "Bystander Effect in Synergy to Anergy in Oral Tolerance of Blomia Tropicalis/Ovalbumin Murine Co-Immunization Model", JOURNAL OF CLINICAL IMMUNOLOGY, PLENUM PUBLISHING CO, US, vol. 25, no. 2, 1 March 2005 (2005-03-01), pages 153-161, XP002618894, ISSN: 0271-9142, DOI: DOI:10.1007/S10875-005-2821-3 cited in the application
- GUSTAVO C RAMOS ET AL: "Cell-mediated immune response to unrelated proteins and unspecific inflammation blocked by orally tolerated proteins", IMMUNOLOGY, BLACKWELL PUBLISHING LTD, vol. 126, no. 3, 1 March 2009 (2009-03-01) , pages 354-362, XP002618895, ISSN: 1365-2567, DOI: DOI:10.1111/J.1365-2567.2008.02901.X cited in the application
- LI-FANG WANG ET AL: "Antigen-driven bystander effect accelerates epicutaneous sensitization with a new protein allergen", JOURNAL OF BIOMEDICAL SCIENCE, KLUWER ACADEMIC PUBLISHERS, DO, vol. 16, 1 March 2009 (2009-03-01), page 28, XP002618896, ISSN: 1423-0127, DOI: DOI:10.1186/1423-0127-16-28 cited in the application
- BRIMNES J ET AL: "Sublingual Immunotherapy (SLIT) Induce Systemic Tolerance In Naive Mice", JOURNAL OF ALLERGY AND CLINICAL IMMUNOLOGY, MOSBY, INC, US, vol. 123, no. 2, 1 February 2009 (2009-02-01), page S127, XP025998652, ISSN: 0091-6749, DOI: DOI:10.1016/J.JACI.2008.12.468 [retrieved on 2009-02-01] cited in the application
- KILDSGAARD J ET AL: "Sublingual immunotherapy (SLIT) in sensitised mice induces mucosal IgA antibodies", JOURNAL OF ALLERGY AND CLINICAL IMMUNOLOGY, MOSBY, INC, US, vol. 115, no. 2, 1 February 2005 (2005-02-01), page S207, XP004845984, ISSN: 0091-6749, DOI: DOI:10.1016/J.JACI.2004.12.839 cited in the application
- WEINER ET AL: "Oral tolerance: immune mechanisms and treatment of autoimmune diseases", IMMUNOLOGY TODAY, ELSEVIER PUBLICATIONS, CAMBRIDGE, GB, vol. 18, no. 7, 1 July 1997 (1997-07-01), pages 335-343, XP022014835, ISSN: 0167-5699, DOI: DOI:10.1016/S0167-5699(97)01053-0 cited in the application
- RASK C ET AL: "Shorter Dosing Intervals of Sublingual Immunotherapy Lead to More Efficacious Treatment in a Mouse Model of Allergic Inflammation", SCANDINAVIAN JOURNAL OF IMMUNOLOGY, BLACKWELL SCIENCE PUBL., OXFORD, GB, vol. 71, no. 6, 1 June 2010 (2010-06-01), pages 403-412, XP002618897, ISSN: 0300-9475, DOI: DOI:10.1111/J.1365-3083.2010.02394.X [retrieved on 2010-03-17] cited in the application
- BRIMNES J ET AL: "Sublingual immunotherapy reduces allergic symptoms in a mouse model of rhinitis", CLINICAL AND EXPERIMENTAL ALLERGY, BLACKWELL SCIENTIFIC PUBLICATIONS, LONDON, GB, vol. 37, no. 4, 1 April 2007 (2007-04-01), pages 488-497, XP002618898, ISSN: 0954-7894, DOI: DOI:10.1111/J.1365-2222.2006.02624.X [retrieved on 2006-11-28] cited in the application
- KILDSGAARD J ET AL: "Sublingual immunotherapy in sensitized mice", ANNALS OF ALLERGY, ASTHMA & IMMUNOLOGY, ARLINGTON HEIGHTS, IL, US, vol. 98, no. 4, 1 April 2007 (2007-04-01), pages 366-372, XP026959766, ISSN: 1081-1206 [retrieved on 2007-04-01] cited in the application
- CATHERINE A SABATOS-PEYTON ET AL: "Antigen-specific immunotherapy of autoimmune and allergic diseases", CURRENT OPINION IN IMMUNOLOGY, ELSEVIER, OXFORD, GB, vol. 22, no. 5, 1 October 2010 (2010-10-01), pages 609-615, XP002618899, ISSN: 0952-7915, DOI: DOI:10.1016/J.COI.2010.08.006 [retrieved on 2010-09-17] cited in the application
- LUND GITTE ET AL: "Effect on Cell Surface Markers following Allergen-Induced Desensitization of Human Whole-Blood Basophils", INTERNATIONAL ARCHIVES OF ALLERGY AND IMMUNOLOGY, vol. 153, no. 4, 2010, pages 323-334, XP002664889, ISSN: 1018-2438
- ALLAM J P ET AL: "Phl p 5 resorption in human oral mucosa leads to dose-dependent and time-dependent allergen binding by oral mucosal Langerhans cells, attenuates their maturation, and enhances their migratory and TGF-beta1 and IL-10-producing properties", JOURNAL OF ALLERGY AND CLINICAL IMMUNOLOGY, MOSBY, INC, US, vol. 126, no. 3, 1 September 2010 (2010-09-01), pages 638-645.E1, XP027399156, ISSN: 0091-6749 [retrieved on 2010-09-01]
- FANTA C ET AL: "SYSTEMIC IMMUNOLOGICAL CHANGES INDUCED BY ADMINISTRATION OF GRASS POLLEN ALLERGENS VIA THE ORAL MUCOSA DURING SUBLINGUAL IMMUNOTHERAPY", INTERNATIONAL ARCHIVES OF ALLERGY AND IMMUNOLOGY, KARGER AG, CH, vol. 120, no. 3, 1 November 1999 (1999-11-01), pages 218-224, XP008044918, ISSN: 1018-2438, DOI: 10.1159/000024270

## Description

### Technical Field

The present invention is within the field of immunotherapy, treatment of hypersensitivity immune responses and bystander suppression.

### Background

Allergen-specific immunotherapy (SIT) was introduced into clinical medicine almost a century ago for the treatment of type I hypersensitivity immune responses and SIT is currently the only treatment leading to prolonged tolerance against allergens. In SIT, the specific allergen or a cross-reacting allergen thereof is repeatedly administered to the individual, usually either by subcutaneous administration or by sublingual administration, during a longer period, usually more than one year. Typically, the patient experiences lower symptom scores on re-exposure to the allergen(s) after some weeks or months treatment (Allergens and Allergen Immunotherapy 4th Ed, 2008, Ed by R Lockey and D Ledford, Informa healthcare)*.*

One challenge encountered with SIT is that the allergen needs to be identified and that the individual undergoing SIT has significant elevated risk of getting severe systemic adverse reactions like anaphylaxis as well as local adverse reactions like swelling and itching. Therefore, there is a need to improve the treatment of a hypersensitivity immune response.

About 20 years ago, Miller et al (1991) demonstrated the principle of providing peripheral tolerance to an antigen (MBP) (myelin basic protein) in naïve rats not yet sensitized to MBP antigen by treatment of the naïve rats by intra-gastric administration of OVA (an antigen unrelated to MBP) before the rats were exposed to MBP for the first time. Miller et al (1991) found that the rats were only protected from developing EAE, if the rats were co-exposed to the unrelated antigen (OVA) along with being exposed to the MBP for the first time.

Later on several researchers have investigated bystander suppression to an immune response by administering an antigen unrelated to the antigen triggering the immune response.

For example, Dahlman-Hoglund et al (1995) have investigated the suppression of an immune response in naïve rats subsequent to feeding the naïve rats orally with a first antigen prior to challenging the naïve rat to a second antigen so as to trigger an inadvertent immune response. The investigators found that the immune response due to the second antigen was suppressed only where the rats were exposed to a mixture containing both the first antigen and the second antigen at the time of triggering the inadvertent immune response. In contrast, suppression of the immune response was not achieved when the first and second antigens were administered at two separate body parts of the rat.

The publication of Millington et al (2004) relates to feeding mice with a soluble protein (OVA) so as to induce bystander suppression to an unrelated antigen.

Oliveira CR et al (2005) have suggested bystander effect of a non-related antigen as an interesting mechanism to control sensitization to a new allergen in individuals already sensitized to one allergen. The authors have indicated that per-oral pretreatment with OVA leads to bystander down regulation of specific antibodies towards the allergen, but only when OVA is present at the subsequent immunization.

The publication of Wang Lifang et al (2009) relates to antigen-driven bystander effect that accelerates epicutaneous sensitization with a new protein allergen. The publication of Brimnes et al (2009) relates to the investigation of sublingual immunotherapy in naïve mice. The publication of Kildsgaard et al (2005) relates to the investigation of sublingual immunotherapy in sensitized mice. The publication of Weiner et al (1997) relates to oral tolerance and treatment of autoimmune diseases. The publication of Rask et al (2010) relate to the investigation of sublingual immunotherapy in a mouse model of allergic inflammation. The publication of Brimnes et al (2007) relates to the investigation of sublingual immunotherapy in a mouse model of rhinitis. The publication of Kildsgaard et al (2007) relates to the investigation of sublingual immunotherapy in sensitized mice.

The international patent application WO 2004/082710 relates to a product comprising i) a modulator of the Notch signalling pathway; and ii) an allergen or allergen bystander antigen or antigenic determinant thereof, or a polynucleotide coding for an allergen or allergen bystander antigen or antigenic determinant thereof; as a combined preparation for simultaneous, contemporaneous, separate or sequential use for modulation of the immune system.

### Summary of the Invention

In contrast to conventional allergen specific immunotherapy where the individual is treated with the specific allergen (incl. a mixture of allergens or cross-reacting allergens thereof) triggering the hypersensitivity immune response, the present invention relates to the treatment, including prophylactic treatment, of a hypersensitivity immune response in an individual with an antigen unrelated to the allergen triggering a hypersensitivity immune response in the individual. That is to say that the present invention also relates to bystander suppression of a hypersensitivity immune response.

The present inventors have provided evidence that a hypersensitivity immune response triggered by exemplary antigens in mice could be significantly reduced if the mice where pre-treated with another antigen unrelated to the "triggering" antigen. Interestingly, the alleviation of the hypersensitivity immune response was also reflected in clinically relevant readouts of an allergic immune response, e.g. reduction in number of sneezes or reduction in airway hyperresponsiveness. Even more interestingly, the alleviation of the hypersensitivity immune response could be demonstrated in mice pre-sensitized to the "triggering" antigen as well as in mice not sensitized to the "triggering" antigen. The inventors have also found that the immune response was only reduceable if the unrelated antigen is available together with the "triggering" antigen at the same time as the mice are challenged/ exposed to the "triggering" antigen.

Thus, it may be understood that the present treatment of an immune response with an antigen unrelated to the "triggering" antigen only is workable, if the target organ of the "triggering" antigen is also exposed to the unrelated antigen at the same time as the "triggering" antigen. For example, it might be envisaged that the treatment with an unrelated antigen of an individual suffering from a hypersensitivity immune response due to exposure to an inhalation allergen is only workable, if the unrelated antigen is also exposed to the respiratory tract of the individual.

As recognized by the present inventors, it is a highly challenging task to ensure co-availability / co-exposure of both the antigen triggering the inadvertent immune response and the unrelated antigen at the organ target for the natural exposure of a "triggering" antigen. At least, it is envisaged that where the target organ is the respiratory tract, it is a challenge to ensure co-exposure of a pollen allergen and an unrelated antigen, unless the individual administers the unrelated antigen directly to the respiratory tract concomitantly with the airborne allergen material (e.g. pollen).

Also interestingly, the inventors have found that bystander suppression is more effective if the unrelated antigen is administered to a mucosa of the oral cavity, such as to a mucosa of the sublingual part of the oral cavity, rather than to a mucosa of the gastro-intestinal tract. To the best knowledge of the present inventors, they are the first to realize that bystander suppression is more efficient if the unrelated antigen is administered to a mucosa of the oral cavity.

Thus, a first aspect of the invention relates to a proteinaceous antigen for use in the treatment of a type 1 hypersensitivity immune response of the respiratory tract in an individual in need thereof, wherein
i) the type 1 hypersensitivity immune response is triggered by an allergen upon the individual's exposure to an environmental source material comprising said allergen;
ii) the proteinaceous antigen is unrelated to the allergen(s) triggering the type 1 hypersensitivity immune response in the individual in the sense that it 1) does not bind IgE antibodies derived from said individual in detectable levels in a RAST test
iii) the proteinaceous antigen is administered in a therapeutically effective amount to the sublingual mucosa of said individual,
iv) the proteinaceous antigen is also administered to the respiratory tract within a period at least coinciding partly or entirely with the individual's exposure to said source material, and
v) the proteinaceous antigen is not co-administered together with said allergen.

In a second aspect, the invention relates to a proteinaceous antigen for use in the treatment of a type 1 hypersensitivity immune response of the gastro-intestinal tract in an individual in need thereof, wherein
i) the immune response is triggered by an allergen upon the individual's exposure to a dietary source material comprising said allergen;
ii) the proteinaceous antigen is unrelated to the allergen(s) triggering the hypersensitivity immune response in the individual in the sense that it 1) does not bind IgE antibodies derived from said individual in detectable levels in a RAST test and/or 2) does not induce a hypersensitivity immune reaction in said individual;
iii) the proteinaceous antigen is administered in a therapeutically effective amount to the sublingual mucosa of said individual,
iv) the proteinaceous antigen is also administered to the gastro-intestinal tract within a period at least coinciding partly or entirely with the individual's exposure to said source material, and
v) the proteinaceous antigen is not co-administered together with said allergen.

In a third aspect, the invention relates to a proteinaceous antigen for use in the treatment of a type 1 hypersensitivity immune response of the skin in an individual in need thereof, wherein
i) the immune response is triggered by an allergen upon the individual's exposure to an environmental source material comprising said allergen;
ii) the proteinaceous antigen is unrelated to the allergen(s) triggering the hypersensitivity immune response in the individual in the sense that it 1) does not bind IgE antibodies derived from said individual in detectable levels in a RAST test and/or 2) does not induce a hypersensitivity immune reaction in said individual;
iii) the proteinaceous antigen is administered in a therapeutically effective amount to the sublingual mucosa of said individual,
iv) the proteinaceous antigen is also administered to the skin within a period at least coinciding partly or entirely with the individual's exposure to said source material, and
v) the proteinaceous antigen is not co-administered together with said allergen.

In a sub aspect thereof, it should be understood that wherein the antigen is for use in treatment of a hypersensitivity immune response in an individual, the individual has specific IgE antibodies, such as specific serum IgE antibodies, against the allergen(s). Moreover, it should be understood that this individual does not necessarily present clinical symptoms of the hypersensitivity immune response at the time of treatment, but the individual is sensitized to an allergen.

As recognized by the present inventors, the requirement of co-availability of both the "triggering" antigen and the unrelated antigen at a target organ can be practiced by various means. In the present invention, co-availability is achieved by by also administering the unrelated antigen to the relevant target organ of allergen exposure, such as by administering the unrelated antigen to the respiratory tract, gastro-intestinal tract or skin, which are recognized as main target organs for environmental allergens via inhalation and contact to skin or for dietary allergens via ingestion of food.

### Legends to Figures

Figure 1. An outline of the prophylactic SLIT treatment protocol with an unrelated antigen in mice.
Figure 2. Panel A represents result from proliferation of spleen cells isolated from Phl p versus buffer SLIT treated mice that have subsequently been i.p. immunized with OVA and Phl p together, whereas panel B shows the proliferation of spleen cells isolated from similar mice subsequently i.p. immunized with OVA, only. Each dot represents an individual mouse.
Figure 3. Panel A and B represent cytokine levels of IL-4 and IL-5, respectively, at day 5 as determined in the supernatant of the spleen cell culture derived from Phl p or buffer SLIT treated mice that have subsequently been i.p. immunized with OVA and Phl p together. Each dot represents an individual mouse.
Figure 4. Panel A represents result from proliferation of spleen cells isolated from Phl p versus buffer SLIT treated mice that have subsequently been i.p. immunized with Phl p and OVA together, whereas panel B shows the proliferation of spleen cells isolated from similar mice subsequently i.p. immunized with Phl p, only.
Figure 5. An outline of the prophylactic SLIT versus per-oral treatment with an unrelated antigen in mice is depicted.
Figure 6. The figure shows the proliferation of spleen cells from mice SLIT treated with Phl p extract either by the sublingual or the per-oral (intragastric gavache) route for two weeks. These treatments were followed by an intra-peritoneal injection with Phl p and OVA together. Following euthanization, the spleen cells were re-stimulated by a low dose of OVA (5µg/mL, panel A) or a high dose of OVA (125 µg/mL, panel B). Each dot represents an individual mouse.
Figure 7. An outline of the prophylactic SLIT treatment with an unrelated antigen in mice with clinically relevant readouts of a Th2-driven immune response is depicted.

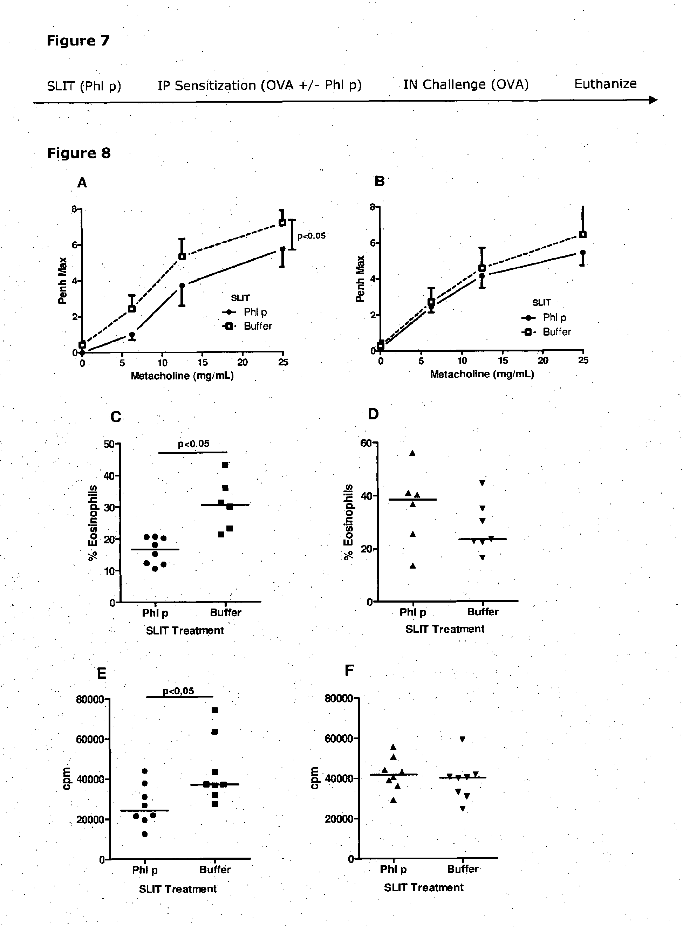

Figure 8. The figure shows the result from prophylactic SLIT treatment of naïve mice with an unrelated antigen prior to immunizing and intra-nasally challenging the mice to another antigen so as to induce clinically relevant readouts of allergic asthma. Panel A and B show the airway hyperresponsiveness in SLIT treated mice immunized with OVA and Phl p together and with OVA only, respectively. Panel C and D show the percentage of eosinophils in bronchoalveolar lavage fluid (BAL) obtained from SLIT treated mice immunized with OVA and Phl p together and with OVA only, respectively. Panels E and F show the in-vitro proliferation of cervical lymph node cells isolated from LIT treated mice immunized with OVA and Phl p together and with OVA only, respectively.
Figure 9. An outline of SLIT treatment with an unrelated antigen (phl p) in OVA antigen pre-sensitized mice developing an immune response against OVA antigen with clinically relevant readouts of a Th2-driven immune response.
Figure 10. Panel A and B show the fraction of eosinophils in the BAL fluid in OVA pre-sensitized mice SLIT treated with Phl p (unrelated antigen) versus mice SLIT treated with buffer, upon the subsequent intranasal challenge with OVA and Phl p together versus OVA alone, respectively.
Figure 11. Levels of IL-5 in BAL fluid obtained from OVA pre-sensitized mice and SLIT phl p treated mice versus SLIT buffer treated mice. Panel A refers to mice exposed to both OVA and Phl p (unrelated antigen) upon intranasal challenge. Panel B refers to mice exposed to OVA (antigen triggering an immune response) upon intranasal challenge.
Figure 12. Airway hyperresponsiveness in OVA pre-sensitized and SLIT phl p treated mice versus SLIT buffer treated mice. Panel A refers to mice exposed to both OVA and Phl p (unrelated antigen) upon intranasal challenge. Panel B refers to mice exposed to OVA (antigen triggering an immune response) upon intranasal challenge.
Figure 13. An outline of SLIT treatment with an unrelated antigen (OVA) in Phl p antigen pre-sensitized mice developing an immune response against Phl p antigens with clinically relevant readouts of a Th2-driven immune response.
Figure 14. Panel A and B show the number of sneezes in Phl p sensitized mice SLIT treated with OVA versus buffer SLIT treated mice upon intranasal challenge with Phl p. Panel A represents mice intra-nasally challenged to both Phl p and OVA and
Panel B represents mice mice intra-nasally challenged to Phl p, only.
Figure 15. Panel A and B show the fraction of eosinophils in the BAL fluid in Phl p pre-sensitized mice SLIT treated with OVA (unrelated antigen) versus mice SLIT treated with buffer, upon the subsequent intranasal challenge with Phl p and OVA together versus Phl p alone, respectively.
Figure 16. Shows the levels of IL-5 in cell culture supernatants of cervical LN cells obtained from Phl p pre-sensitized mice and SLIT OVA treated mice versus SLIT buffer treated mice.
Figure 17. An outline of SLIT treatment with an unrelated antigen (phl p) in OVA antigen pre-sensitized mice developing an immune response against Phl p antigens with clinically relevant readouts of a Th2-driven immune response.
Figure 18. Panel A shows airway hyper responsiveness in OVA pre-sensitized and SLIT phl p treated mice (open circles) versus SLIT buffer treated mice (open triangles). Panel B shows the fraction of eosinophils in BAL fluid in OVA pre-sensitized and SLIT phl p treated mice (filled circles) versus SLIT buffer treated mice (filled triangles). Panel C shows in vitro proliferation of spleen cells from OVA pre-sensitized and SLIT phl p treated mice (filled circles) versus SLIT buffer treated mice (filled triangles)
Figure 19. Panel A shows the fraction of eosinophils in BAL fluid in mice that were SLIT treated with either Phl p 5 or buffer and subsequently i.p. sensitized with OVA and Phl p 5 together and then intranasally challenged with OVA. Panel B shows the in vitro proliferation of spleen cells from the same mice as above upon re-stimulation with OVA.
Figure 20. CIE diagram showing precipitates of serum IgE antibodies and antigens of a grass pollen extract of Pleum Pratense (radioactive + non-radioactive).
Figure 21. Allogram showing the number of individuals having various antigens (allergens) of a grass pollen extract of Pleum Pratense detected in their serum. Where, a precipate cannot be assigned to any of the scores; strong, moderate or weak, there is an empty space in the Allergogram. Empty spaces designate an unrelated antigen.
Figure 22 CIE diagram showing precipitates of serum IgE antibodies and antigens of a mite allergen extract of Dermatophagoides pteronyssinus (Der p) (radioactive + non-radioactive).

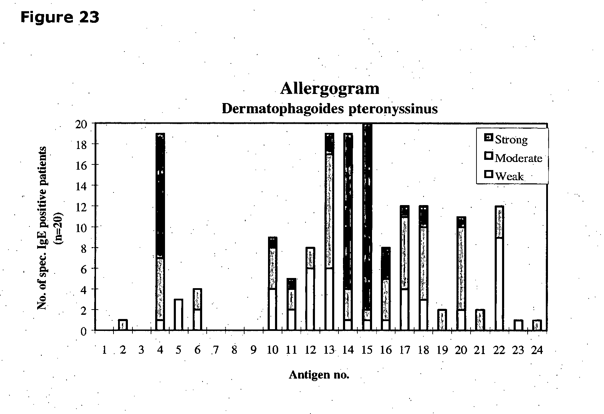

Figure 23. Allogram showing the number of individuals having various numbered antigens (allergens) of Dermatophagoides pteronyssinus (Der p) detected in their serum.
Figure 24. CIE diagram showing precipitates of serum IgE antibodies and antigens of a mite allergen extract of Dermatophagoides farinae (Der f) (radioactive + non-radioactive).

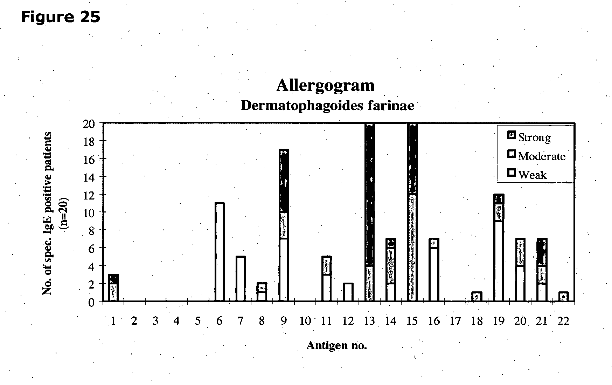

Figure 25. Allogram showing the number of individuals having various numbered antigens (allergens) of Dermatophagoides farinae (Der f) detected in their serum.

### Detailed Description

### Definitions

The following terms and phrases shall have the following meaning;
The term "a" or "an" refers to an indefinite number and shall not only be interpreted as "one" but also be interpreted in the meaning "some" or "several".
The phrase "an allergen" shall be interpreted as one or more allergen(s).
The term "allergen" is meant to designate an allergen capable of eliciting a hypersensitivity immune response in an individual, such as in an animal, such as in a human. The allergen may be a sensitizing allergen or a cross-reacting allergen.
The term "sensitizing allergen" is meant to designate an allergen capable of triggering the immune system to produce IgE antibodies in an individual.
The term "cross-reacting allergen" defines an allergen that responds to IgE antibodies originally created against a sensitizing allergen. That is to say that a cross-reacting allergen is an allergen capable of eliciting a hypersensitivity immune response due to cross-reactive IgE antibodies induced by a sensitizing allergen. For example the allergen Mal d 1 of apples is a protein homologous to Bet v 1 of birch tree pollen, and able to elicit a hypersensitivity immune response in an individual pre-sensitized to bet v 1 allergen due to its ability to trigger mast cells loaded with IgE anti-Bet v 1.
The term "major allergen" shall mean an allergen that causes sensitization in more than 50% of a population of patients having a hypersensitivity immune response triggered by exposure to an allergen source material comprising said allergen.
The term "minor allergen" shall mean an allergen that causes sensitization in less than 50% of a population of patients having a hypersensitivity immune response triggered by exposure to an allergen source material comprising said allergen.
The phrase "antigen unrelated to an allergen" and the term "unrelated antigen" are interchangeable terms and are meant to designate a proteineous antigen distinct and different from an allergen, such as different from a sensitizing allergen as well as a cross-reacting allergen.
The phrase "antigen unrelated to an allergen triggering a hypersensitivity immune response in an individual" is meant to designate a proteineous antigen distinct and different from an allergen, such as different from a sensitizing allergen as well as a cross-reacting allergen capable of triggering a hypersensitivity immune response in the individual.
The phrase "antigen obtainable from a source material" is meant to designate that the antigen is isolated from the source material comprising the allergen or is otherwise isolated/obtained from another source material, but still identical to or at least essentially identical to the unrelated antigen present in the original source material comprising the allergen capable of triggering a hypersensitivity immune response in the individual.
The phrase "antigen derivable from a source material" is meant to designate an antigen identical to or at least essentially identical to the unrelated antigen present in the source material comprising the allergen capable of triggering a hypersensitivity immune response in an individual in need thereof, but reproduced by means of chemical and/or biological methods, such as by peptide synthesis and/or recombinant protein techniques according to methods well known in the art of proteins.
The term "antigen essentially identical to the unrelated antigen" is meant to designate that the antigen is slightly modified in relation to the unrelated molecule that is present or isolated from the source material comprising the allergen. Typical modifications are defined further herein.
The term "bystander suppression" is generally meant to encompass the ability to suppress an immune reaction in an individual towards one antigen (A) by treatment of the individual with another unrelated antigen (B).
The term "tolerance-induced bystander suppression" is generally meant to encompass the ability to suppress an immune reaction in an individual towards one antigen (A) by treatment of the individual with another unrelated antigen (B).
The term "co-availability" as used herein is meant to designate that the unrelated antigen is presented to the same target organ, such as to the same mucosa or skin, as reached by the "triggering" allergen through the natural exposure of a source material comprising the allergen. It does not necessarily mean that the unrelated antigen and the allergen are available or presented to the target organ at the exactly same time, but close enough in time to ensure that bystander suppression takes place. For example, the unrelated antigen is presented to/available at the target organ hours, days or weeks before, or after, the "triggering" allergen reaches the target organ. However, the unrelated antigen is preferably presented to the target organ within a period at least overlapping partly or entirely, such as coinciding partly or entirely, with the period where the triggering allergen is exposed to the same target organ.
The term "co-exposure" as used herein, is meant to encompass that the disease-eliciting antigen, such as the "triggering" allergen, and the unrelated antigen is exposed to the same target organ, e.g. the same mucosa upon the time where the individual is exposed to "triggering" allergen. It does not necessarily mean that the unrelated antigen and the sensitizing allergen are available or presented to the same body part at the same time, but close enough in time, to ensure that the bystander suppression takes place. For example, the unrelated antigen is presented to/available at the target organ hours, days or weeks before, or after, the "triggering" allergen reaches the target organ. However, the unrelated antigen is preferably presented to the target organ within a period at least overlapping partly or entirely, such as coinciding partly or entirely, with the period where the triggering allergen is exposed to the same target organ.
The term "immunotherapy" is meant to encompass therapy, wherein the therapeutically active agent (the immunomodulator) is an antigen. Immunotherapy usually encompass repeatedly administration of a sufficient dose of the antigen, usually in microgram quantities, over a prolonged period of time, usually for months or for years, wherein the antigen is administered daily, weekly, bi-weekly, or monthly.
The term "an individual" is meant to designate a mammal having an adaptive immune system, such as a human, a pet like a dog or a cat, and a farm animal like a horse or cattle.
The phrase "an individual in need of thereof" is meant to encompass an individual having a hypersensitivity immune response, an individual sensitized to an allergen as well as an individual in risk of being sensitized to an allergen or in risk of developing a hypersensitivity immune response. The individual may present clinically symptoms of a hypersensitivity immune response or the individual may only be sensitized to an allergen and not yet presenting clinically symptoms of a hypersensitivity immune response. An individual in risk of being sensitized to an allergen may be identified due to family histories of atopic individuals.
The phrase "an individual sensitized to an allergen" is meant to encompass an individual having specific IgE antibodies, such as serum antibodies, towards the allergen.
The phrase "prophylactic treatment" is meant to encompass treatment, such as by immunotherapy, of an individual with the aim to alleviate a hypersensitivity immune response or completely preventing the individual from developing a hypersensitivity immune response. Prophylactic treatment, such as prophylactic immunotherapy, is therefore initiated before the individual becomes sensitized to an allergen. This may be realized by initiating immunotherapy before the individual has raised detectable serum IgE antibodies capable of binding specifically to the sensitizing allergen or that any other biochemical marker indicative of a hypersensitivity immune response can be detected in biological samples isolated from the individual. Furthermore, prophylactic immunotherapy shall also designate immunotherapy initiated before the individual has evolved clinical symptoms of the disease, such as symptoms of allergic rhinitis or allergic asthma.
The term "treatment" refer to any type of treatment or prevention that imparts a benefit to a subject afflicted with or at risk of developing a hypersensitivity immune response to an allergen of interest, including improvement in the condition of the subject (e.g., in one or more symptoms), delay in the onset of symptoms or slowing the progression of symptoms, etc. As used herein, "treatment" is not necessarily meant to imply cure or complete abolition of symptoms, but refers to any type of treatment that imparts a benefit to a patient.
The phrase "therapeutically effective amount" is meant to designate an amount effective to treat, such as an amount sufficient to achieve the desirable effect. For example, a therapeutically effective amount is the total dose of an unrelated antigen administered during a period of immunotherapy in order to achieve the intended efficacy or the maximal dose tolerated within a give period. The total dose may be divided into single doses administered daily, twice a week, weekly, every second or fourth week or monthly depending on the route of administration. The total dose may be administered in different concentrations. It is expected that a single dose is in the microgram range, such as in the range of 5 to 500 microgram dependent on the unrelated antigen.
The phrase "sensitized to an allergen" is generally meant to encompass that the individual has been exposed to an allergen in a manner that the individual's adaptive immune system displays memory to the allergen, such as has raised detectable IgE antibodies against the allergen or that t-cells stimulated in-vitro are able to proliferate under the presence of the sensitizing allergen.
The term "sublingual route" and the term "sublingual administration" are interchangeable terms meant to designate that the unrelated antigen is administered topically or trans-mucosally to the sublingual mucosa of an individual. Preferably, the unrelated antigen is provided as a pharmaceutical formulation suitable for this purpose, such as a tablet, e.g. fast-disintegrating tablet, or as a solution for drop-wise administration.
The term "adjuvant" refers to a substance that enhances the immune response to an antigen. Depending on the nature of the adjuvant it can promote either a cell-mediated immune response, humoral immune response or a mixture of the two.

### Summary on data provided herein

As shown in the Example section herein, the present inventors have studied bystander suppression by use of various experimental designs of murine models.

One of the experimental designs is a prophylactic murine model and is used in Examples 1 and 2. This model allows for investigating if prophylactic treatment with one antigen is able to down regulate a systemic inflammatory immune response caused by another antigen (triggering antigen) to which the first antigen is unrelated to. Moreover, this model allows for investigating if the down regulation is dependent on the presence of the unrelated antigen at the systemic challenge.

As a first step, naïve mice receive sublingual immunotherapy (SLIT) with the unrelated antigen in order to induce tolerance. Subsequently, the effect of the SLIT treatment is evaluated by inducing a systemic immune response by injecting the "triggering" antigen (i.e. allergen) adsorbed to alum intraperitoneally (i.p.). The immune response thus generated is generally characterized by spleenic T-cell proliferation and production of Th2 cytokines upon *in vitro* re-stimulation with the allergen. Therefore, the immune response generated in this simple prophylactic murine model reflects the human situation, where the individual undergoes prophylactic immunotherapy with the aim of preventing sensitization to a Th2-inducing allergen and/or preventing an immune response triggered by a Th2-inducing allergen.

To study bystander suppression, one group of mice is challenged with both the allergen and the unrelated antigen at the i.p. injection. Bystander suppression will be obtained in the mice, if splenic T-cells derived from mice treated sublingually with the unrelated antigen show reduced T-cell proliferation upon in vitro re-stimulation with the Th2-inducing allergen compared to T-cells derived from mice treated sublingually with placebo (buffer). In the current examples, two single antigens, OVA and Phleum p 5 allergen of grass pollen as well as a mixture of Pheum p allergens (aqueous allergen extract of grass pollen of the species Phleum Pratense) have been used as exemplary unrelated antigens. Two different Th2-inducing allergens, OVA and Phl p extract, have been used as exemplary allergens in triggering a Th2-like immune response in the murine models. For example, where OVA is applied as the unrelated antigen, the mice are challenged with the Phl p allergen extract, and vice versa with OVA as the challenge allergen.

Another experimental design of the prophylactive murine model is adopted in example 3 and 6 with the aim of demonstrating bystander suppression of airway inflammation and thus demonstrating bystander suppression of a clinically relevant immune response, as clinically relevant parameters like the fraction of eosinophils in the lung and airway hyper-reactivity are assessed. In this model, the i.p. injection that follows after the prophylactic SLIT treatment can be regarded as a sensitizing step, because it is followed by an intranasal challenge with the same allergen used at the i.p. sensitizing step. The additional step of intranasal challenge solely serves to induce airway inflammation clinically relevant to allergic asthma and rhinitis.

To study bystander suppression, one group of mice is challenged to both the allergen and the unrelated antigen at the i.p. injection. Bystander suppression will be obtained in mice sublingually treated with the unrelated antigen, if the mice show reduced eosinophilia and/or reduced airway hyper-reactivity compared to mice treated sublingually with placebo (buffer).

An experimental design of a therapeutic model mice model is used in Examples 4 and 5. The mice are first sensitized to the allergen by i.p. injection and subsequently treated by sublingual immunotherapy with an unrelated antigen. To study bystander suppression of a clinically relevant immune response, the mice are then challenged by intranasal installation of the allergen, either alone or together with the unrelated antigen with the aim of developing airway inflammation as may be observed by increased fraction of eosinophils in the lung, increased airway hyper-reactivity or increased sneezing, all relevant clinical symptoms of allergic asthma and rhinitis. Bystander suppression will be obtained in mice sublingually treated with the unrelated antigen, if the mice show reduced eosinophilia and/or reduced airway hyper-reactivity or sneezing compared to mice treated sublingually with placebo (buffer).

The murine models used herein are well known methods used in the field of studying specific allergen immunotherapy (J Brimnes et al, Clinical and Experimental Allergy 37, 488-497, 2007) and are models reasonably predictive for the efficacy in treatment of humans having the same immunological and clinically readouts as presented in the murine models used herein. Notably, the murine model presents many of the clinical and immunological features associated with human allergic rhinitis and asthma, including sneezing, increased airway hyperreactivity, influx of eosinophils as well as elevated levels of IL-5 and antigen specific IgE both locally and systemically (p 489 of J Brimnes et al 2007). The validity of the murine model of Brimnes et al 2007 has been confirmed in the Editorial of the publication in which the Brimnes article appeared, cf. "New allergy intervention strategies: hitting the mucosal road", U Wiederrnann et aI., Clinical and Experimental Allergy 37, 473-475, 2007.

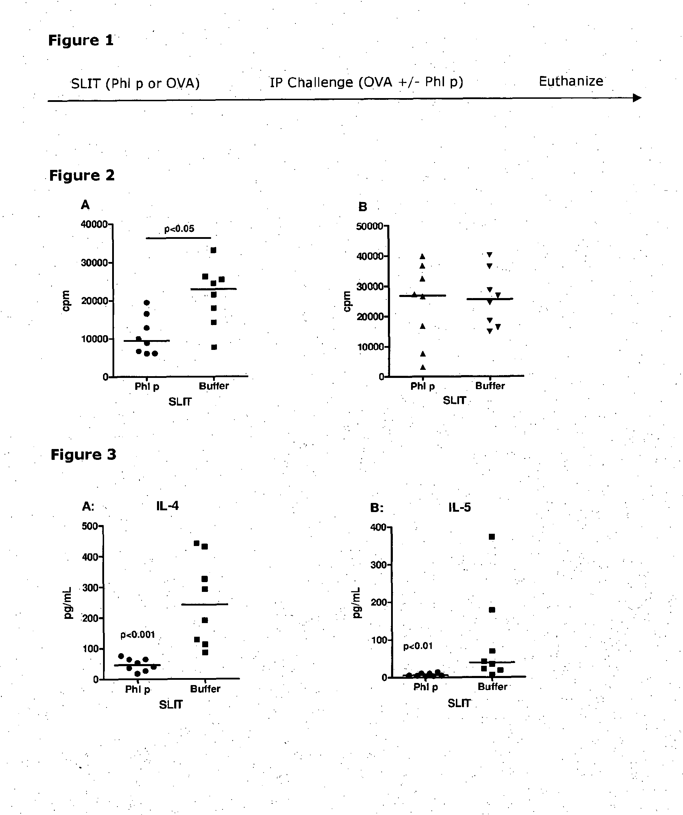

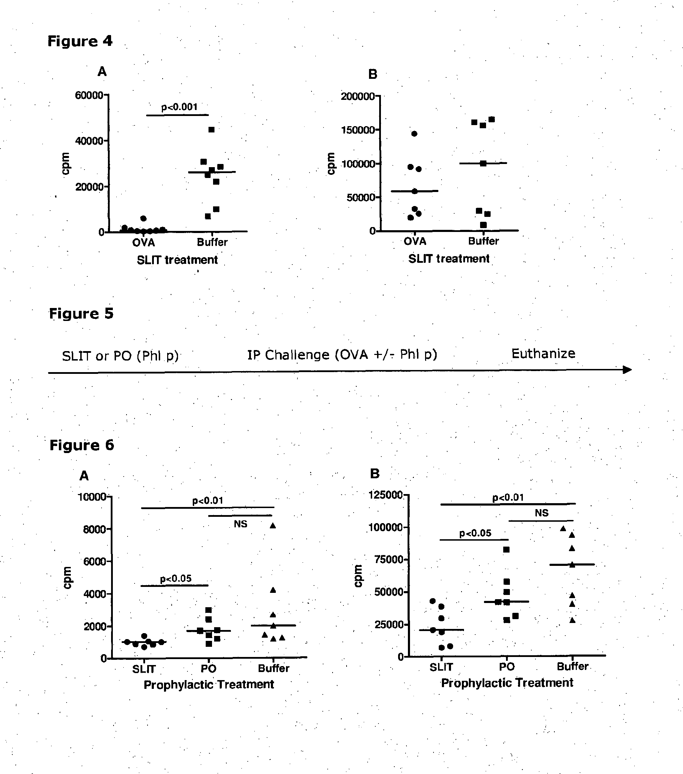

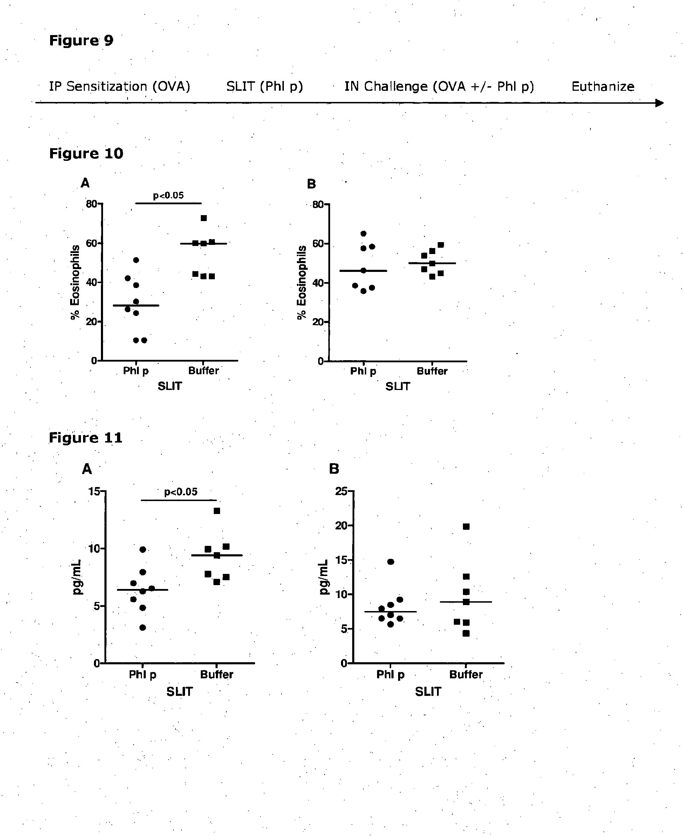

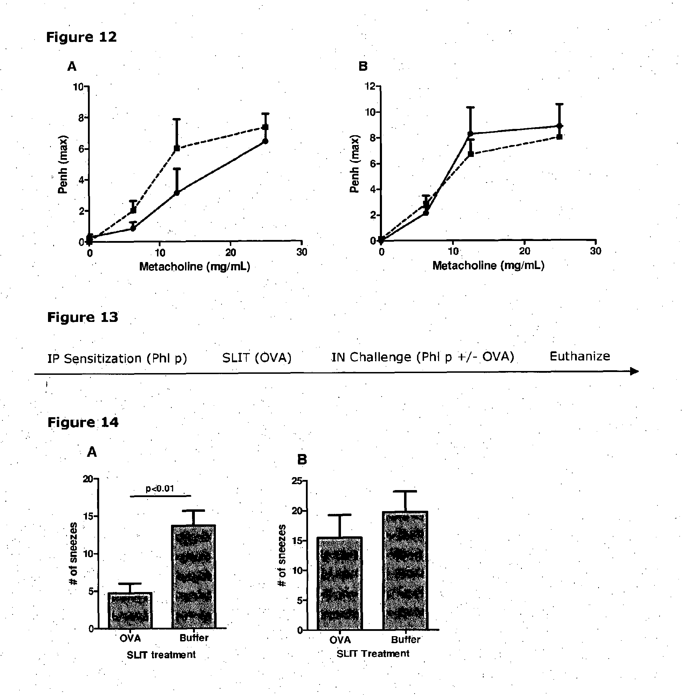

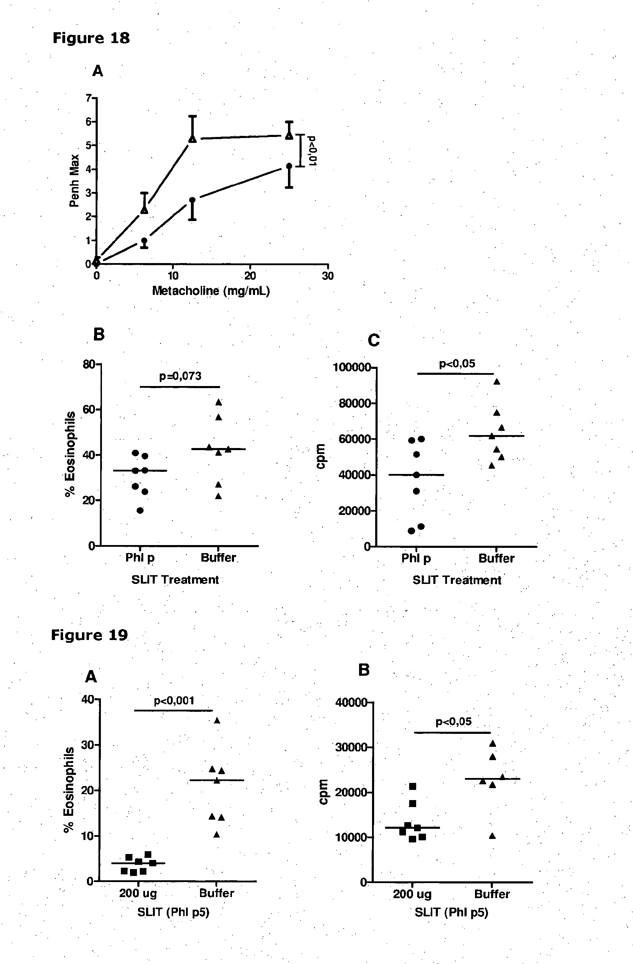

The results presented herein may be summarized as follows: Figures 2A and 3A shows bystander suppression of T-cell proliferation and of the Th2 cytokine Il-4 generation, respectively, in the prophylactic murine model using OVA as the "triggering allergen" and Phl p as the unrelated antigen. Figure 4A shows bystander suppression of T-cell proliferation in the prophylactic murine model using Phl p extract as the "triggering allergen" and OVA as the unrelated antigen. Figures 5A and B both show improved bystander suppression of T-cell proliferation rate in mice sublingually treated with unrelated antigen versus mice per-orally treated with unrelated antigen. The difference between A and B is the dose of OVA used in re-stimulating the spleen T-cells. Figures 8A, C and E show bystander suppression of airway hyperreactivity, influx of eosinophils and T-cell proliferation rate, respectively in the prophylactic murine model using OVA as the "triggering allergen" and Phl p extract as the unrelated antigen. Figures 10A, 11A and 12A show bystander suppression of the influx of eosinophils, IL-5 cytokine levels and airway hyper responsiveness in the therapeutic murine model using OVA as the "triggering" allergen and Phlp extract as the unrelated antigen. Figures 14A, 15A and 16 show bystander suppression with respect to the number of sneezes, influx of eosinophils and levels of IL-5 cytokine, respectively, in the therapeutic murine model using "Phl p extract" as the "triggering" allergen and OVA as the unrelated antigen. Figure 18A, B and C show bystander suppression of airway hyper responsesiveness, influx of eosinophils in BAL and T-cell proliferation rate, respectively, in the prophylactic murine model using OVA as the "triggering allergen" and Phl p extract as the unrelated antigen. Figure 19A and B show bystander suppression of influx of eosinophils in BAL and T-cell proliferation rate, respectively, in the prophylactic murine model using OVA as the "triggering allergen" and Phl p 5 (single antigen) as the unrelated antigen.

As may be noted, bystander suppression could only be demonstrated where the mice also are challenged to the unrelated antigen when challenged to the "triggering allergen". Therefore, figures 2B, 3B, 8B, 8D, 8F, 10B, 11B, 12B, 14B and 15B does not show bystander suppression as these figures refers to mice only challenged to the "triggering" allergen. Also it might be noted that an antigen usually defined as an allergen (Phl p 5 or Phl p extract) is presented herein as exemplary unrelated antigens. However, such allergens are still exemplary unrelated antigens in the sense that the mice are not sensitized to those allergens, but sensitized to OVA in the experiments wherein Phl p 5 or Phl p extract are used as the unrelated antigen.

Thus, the present inventors have provided numerous data pointing to the fact that an antigen unrelated to the "triggering" allergen is able to provide bystander suppression of a hypersensitivity immune response, such as airway inflammation. Furthermore, the numerous data provided herein indicates that bystander suppression also is achievable by use of other not yet tested unrelated antigens and that bystander suppression also can be demonstrated with respect to hypersensitivity immune response of the gastro-intestinal tract (food allergy) or of the skin (atopic dermatitis or contact dermatitis). Bystander suppression of a hypersensitivity immune response triggered by a dietary allergen, like an allergen of peanut, milk, egg or soyabean, may be studied using the murine model outlined in Example 8 and bystander suppression of a hypersensitivity immune response triggered by an allergen associated with contact dermatitis or atopic dermatitis may be studied using the murine model outlined in Example 9.

### Unrelated antigens

As mentioned, uses and methods of the present invention encompasses the administration of an antigen unrelated to the allergen (i.e. the one or more allergen(s)) triggering a hypersensitivity response to the mucosa of the oral cavity as well as to the target organ for the exposure to the "triggering" allergen.

In principle any antigen can candidate as an unrelated antigen of the present invention as long as the unrelated antigen is different from and distinct to the one or more allergen(s) triggering a hypersensitivity immune response in the particular individual in need of treatment thereof and provided that co-availability of the "triggering" allergen and the unrelated antigen at the target organ is achieved. Therefore, an unrelated antigen of the present invention might be identified by determining whether the unrelated antigen can or cannot, at least in detectable levels, bind to IgE antibodies, such as serum IgE antibodies, obtained from the individual to be treated. Further criteria for the selection of a proper unrelated antigen for use in the present therapy may include that the unrelated cannot, at least in detectable levels, bind to IgE antibodies, such as serum IgE antibodies, obtained from a group of individuals that has specific IgE antibodies, such as specific serum IgE antibodies against an allergen of a pre-selected source material of interest, e.g. grass pollen or pollen of various species and genus.

Therefore, in preferred embodiments of the invention, the antigen is unrelated to the "triggering" allergen(s) in the sense that the antigen cannot, at least in detectable levels, bind to IgE antibodies, such as serum antibodies, obtained from a group of individuals having specific IgE antibodies, such as serum IgE antibodies against said allergen(s).

Specific IgE antibodies against an allergen may be tested by well known methods in the art, such as by use of the RAST test that is a radioimmunoassay test to detect specific IgE antibodies against suspected or known allergens. In such test the suspected allergen is bound to an insoluble material and the patient's serum is added. If the serum contains antibodies to the allergen, those antibodies will bind to the allergen. Radiolabeled anti-human IgE antibody is added where it binds to those IgE antibodies already bound to the insoluble material. The unbound anti-human IgE antibodies are washed away. The amount of radioactivity is proportional to the serum IgE for the allergen.In recent years a more superior test named the ImmunoCAP Specific IgE blood test, which in the literature may also be describe as: CAP RAST, CAP FEIA (fluorenzymeimmunoassay), and Pharmacia CAP. The quantitatively detection limit of such test may be as low as about 0.1kU/l. For use in the context of the present invention, the quantitative detection limit may be below 1kU/l, such as below 0.5 kU/l, such as below 0.3kU/l where the ImmunoCAP® Specific IgE blood test or a comparable test is used.

Preferably, the antigen is unrelated to said allergen(s) in the sense that the individual in need of treatment and/or the group of individuals is/are tested negative for specific IgE antibodies, such as serum IgE antibodies, capable of binding to the antigen. That is to say that the IgE antibodies are at least not detectable or at least not present in quantifiable levels.

Obviously, the antigen might also differentiate from the allergen(s) in the sense that the antigen cannot induce a hypersensitivity immune reaction in the individual in need of treatment and/or the group of individuals. As such, in some embodiments of the invention, the antigen is unrelated to the allergen(s) in the sense that the antigen cannot initiate an immediate skin reaction in the individual in need of treatment and/or the group of individuals upon conducting skin prick testing with various concentrations of the antigen.

Also in still other further embodiments of the invention, the antigen is unrelated to the allergen(s) in the sense that the antigen cannot induce histamine release in an in-vitro basophil/mast cell assay using blood from the individual to be treated or in from a group of individuals.

As mentioned, in some embodiments of the invention, the antigen is unrelated to an allergen defined by the IgE antibodies obtained from a group of individuals rather than from the individual to be treated. A representative amount of such a group may vary. It is though anticipated that a group of individuals consist of a number of individuals of at least 4 individuals, such as of at least 5, 10, 15, 20, 30, 40, 50 or at least 100 individuals. Where it is desirable to select an antigen for use in treatment of a greater group of individuals, the antigen might be different from all sensitizing allergens or cross-reacting allergens thereof to which a group of patients have specific IgE antibodies raised against. Here it might be relevant to test a group of patients of at least 30 patients, more preferably 40, even more preferably 50 individuals. Thus, in interesting embodiments of the invention, a group of individuals have a number of individuals in the range of 30 to 1000 individuals, such as of 30 to 500, such as of 30 to 400, such as of 30 to 300, such as of 30 to 250, such as of 30 to 100.

Also, an unrelated antigen may be identified by determining whether the antigen has a distinct antigenic determinant in relation to the allergen, such as sharing B-cell and/or T-cell epitopes, including conformational epitopes and linear epitopes, with the allergen in question. Therefore, in preferable embodiments of the invention, the antigen is unrelated to said allergen(s) in the sense that the antigen does not share any B-cell or T-cell epitopes with said allergen(s). Epitopes can be "mapped," the process of identifying and characterizing the minimum molecular structures that are able to be recognized by the immune system, according to methods known in the art, e.g. protein microarrays, ELISPOT or ELISA techniques, etc. In some embodiments, T cell epitopes which bind to MHC class II molecules are mapped with the use of Tetramer Guided Epitope Mapping (TGEM). See U.S. Patent No. 7,094,555 to Kwok et al... Epitopes may also be predicted based upon computer modeling, e.g., the TEPITOPE program. See, e.g., Kwok et al., Trends in Immunology, 2001, 22(11): 583-588.

Therefore, in preferable embodiments of the invention, the unrelated antigen has an amino acid sequence having less than 70% identity, preferable less than 65, 60, such as less than 65, less than 60%, less than 55%, such as less than 50%, such as less than 45%, such as less than 40% identity, such as less than 30%, such as less than 25%, such as less than 20% identity to the amino acid sequence of the allergen.

Furthermore, an unrelated antigen for use in the present invention may preferable not, such as must not bind to IgE antibodies, such as serum IgE antibodies, obtained from the individual to be treated.

Also the unrelated antigen does not bind, at least not in detectable levels, to IgG antibodies, e.g. rabbit anti IgG, raised after immunization of an animal with one or more or all allergen(s) of a pre-selected source material.

As a proteineous antigen, the unrelated antigen inherently possesses immunogenic properties and is recognized by the individual's immune system as foreign. Thus, an unrelated antigen is capable of inducing specific IgG antibodies, such as IgG₄ or IgGi antibodies, IgM or IgA antibodies capable of binding specifically to the unrelated antigen depending on the route of exposure/administration.

The unrelated antigen is a protein by nature, including a glycoprotein, a lipoprotein and post-translational derivatives thereof e.g. phospho-proteins. The unrelated antigen may also be provided as an immunogenic peptide, including a glycopeptide, a lipopeptide and post-translational derivatives thereof e.g. phospho-peptides, provided that this peptide is not identical or homologous to a peptide fragment of an allergen.

Also, in more preferred embodiments of the invention, the unrelated antigen is a water-soluble antigen. In still more interestingly embodiments of the invention, the unrelated antigen is extractable from an aqueous solution having pH in the range of 6 to 8, such as particularly in the range of 6.5 to 7.5, optionally with the presence of saline and/or presence of relevant enzymes. For example, in interesting embodiments of the present invention, the antigen is extractable from an aqueous solution, such as an aqueous saline solution having pH in the range of 6 to 8, such as preferably in the range of 6.5 to 7.5 for a period not exceeding 60 minutes, such as preferably not exceeding 45, 30, 20, 15, 10 or 6 minutes.

According to the invention, the antigen of the invention is unrelated to the allergen triggering the hypersensitivity immune response. Generally, not all of the allergens of an allergen-containing source material are able to trigger a hypersensitivity immune response. Usually, only major allergens are involved. That is to say that in more interesting embodiments of the invention, the antigen of the invention is unrelated to major allergens of one or more allergen-containing source materials. For example, it might be advantageous to select an unrelated antigen that is unrelated to an allergen, such as particularly an allergen present in an allergen-containing environmental source material or an allergen-containing dietary source material.

### Sources of unrelated antigens

An unrelated antigen may be found in various source materials including those source materials comprising the one or more allergen(s) capable of triggering a hypersensitivity immune response in an individual in need of treatment or in a group of individuals having specific IgE antibodies, such as serum IgE antibodies, against one or more allergens of a pre-selected source material. However, preferably, the unrelated antigen cannot be obtained or derived from an allergen-containing source material comprising the one or more allergen(s) capable of triggering a hypersensitivity immune response in an individual in need of treatment or in a group of individuals.

Such allergen-containing source material may be naturally occurring source materials like an environmental source material (e.g. pollen, animal dander, venoms and latex) or a dietary source material (e.g. nuts and cereals).

An unrelated antigen found in a source material may be identified by use of well known protein separation techniques bearing in mind that proteins are easily denatured and the three-dimensional structure is easily lost. First of all, the source material comprising the allergen shall be identified. The source material may then be subjected to various extraction protocols, preferably where focus is on isolating a water-soluble unrelated antigen. For example, the unrelated antigen may be extracted from the source material comprising mainly aqueous extraction solvents. That is to say that the unrelated antigen is present in an aqueous extraction fraction of the source material, such as being eluted in an aqueous extraction solvent during chromatographic extraction and purification. Lipid extraction solvents may still be used for the purpose of defatting the source material or removing undesirable matter. Various fractions from the extraction procedure may then be subjected to methods suitable for detecting the unrelated antigen. Crossed radio-immuno-electrophoresis (CRIE) is a suitable method for this purpose. In brief, a suitable method for providing an unrelated antigen is to provide water-soluble protein extracts of a source material, e.g. pollen, which are then separated by electrophoresis, followed by another electrophoresis through a gel containing IgG antibodies raised against the water-soluble protein extract in question. IgG antibodies can be raised by immunising rabbits. The resulting antibody: antigen complexes (Ab:Ag complexes) can then be visualized by Coomasie blue staining or be incubated with sera from pollen sensitized individuals followed by anti human IgE antibodies, in order to visualize the allergens of the pollen extract. The antigens that are not bound by IgE antibodies from patient's sera are considered to be candidates to an unrelated antigen.

Example 7 herein relates to the detection of unrelated antigens in three difference source materials, namely in grass pollen and in two different species of house dust mites. As noted, several candidates can be detected by means of the procedure outline above.

As mentioned, the unrelated antigen is preferably not derived from an allergen-containing source material, but it should be understood that an unrelated antigen of the present invention may in some embodiments be obtainable from an allergen-containing source material.

Typical examples of allergen-containing source material are among others:
- Environmental source material or a dietary source material derived from the major taxonomic groups selected from the group consisting of Animalia Arthropoda, Animalia Chordata, Funghi Ascomycetes, Plantae Corniferopsida Plantae Liliopsida and Plantae Magnoliopsida;
- Environmental source material or a dietary source material derived from a genus belonging to the order selected from the group consisting of Astigmata, Diptera, Hymenoptera, Carminora, Perissodactyla, Capnodialis; Eurotialis, Hypocreales, Pleosporales, Saccharomycetes, Corniferales, Poales, Asterales, Fagales, Hevea, Lamiales and Proteales;
- mite crops and mite feces of the major taxonomic group Animalia Arthropoda, dander, hair, saliva, stools or other secretes derived from the major taxonomic group Animalia Chordata; spores or particles derived from the major taxonomic group Funghi Ascomycetes, pollen derived from the major taxonomic group Corniferopsida, pollen derived from the major taxonomic group Plantae Magnoliopsida, pollen derived from the major taxonomic group Plantae Liliopsidae; venoms or secretes derived from the major taxonomic group Animalia Arthropoda; gums or products comprising such a gum derived from trees of the major taxonomic group Plantae Magnoliopsidae;
- shrimps or a shrimp-containing food of the major taxonomic group Animalia Arthropoda; lobster or a lobster-containing food of the major taxonomic group Animalia Arthropoda; fruit, legumes, cereals or beans derived from the major taxonomic group Plantae Liliopsidae or a food product comprising such fruits, legumes, cereals and/or beans; fruit, legumes, cereals or beans derived from the major taxonomic group Plantae Magnoliopsidae or a food product comprising such fruits, legumes, cereals and/or beans; nut or a nut-containing food of the from the major taxonomic group Plantae Magnoliopsidae;
- cow's milk, cow's milk-containing food, chicken egg white, chicken egg white-containing food, fish or fish-containing food;
- crop and/or feces of a mite selected from the group consisting of mites of the genus Acarus, Glycyphagus, Lepidoglyphus, Tyrophagus, Blomia, Dermatophagoides, Euroglyphus, Blatella and Periplaneta;
- dander, hair, saliva or stools of an animal of the genus canis, felis or Equus;
- yeast, mould or funghi of the genus selected from the group consisting of Cladosporium, Aspergillus, Penicillium, Alternaria and Candida;
- pollen of the major taxonomic group Corniferopsida, Plantae Liliopsida or Plantae Magnoliopsida;
- pollen of the genus selected from the group consisting of Chamaecyparis, Cryptomeria, Cupressus, Juniperus, Anthoxanthum, Cynodon, Dactylis, Festuca, Holcus, Hordeum, Lolium, Oryza, Paspalum, Phalaris, Phleum, Poa, Secale, Sorghum, Triticum, Zea, Ambrosia, Artemisia, Alnus, Betula, Corylus, Fraxinus, Olea and Platanus;
- venom of a bee of the genus selected from the group consisting of Apis Bombus, Dolichovespula, Polistes, Polybia, Vespa and Vespula;
- gum or products comprising such a gum derived from the genus Hevea;
- dietary source material (food ingredient or a product comprising such a food ingredient) is derived from a genus selected from the group consisting of Hordeum, Oryza, Secale, Triticum, Zea, Arachis, Corylis, Juglans, Prunus, Anacardium, Pistacia and Glycine;
- dietary source material (food ingredient or a product comprising such a food ingredient) derived from a genus selected from the group consisting of Hordeum, Oryza, Secale, Triticum, Zea, Arachis, Corylis, Juglans, Prunus, Anacardium, Pistacia and Glycine; and/or
- dietary source material derived (food ingredient or a product comprising such a food ingredient) from the group consisting of cow's milk, milk-containing food, chicken egg white, chicken egg white-containing food, fish or fish-containing food.

More over, the unrelated antigen is unrelated to one or more of all of the allergens mentioned below:
- Aca s 13, Gly d 2, Lep d 2, Lep d 5, Lep d 7, Lep d 10, Lep d 13, Tyr p 2, Tyr p 3, Tyr p 10, Tyr p 13, Tyr p 24, Blo t 1, Blo t 2, Blo t 3, Blo t 4, Blo t 5, Blo t 6, Blo t 10, Blo t 11; Blo t 12, Blo t 13, Blo t 19, Blo t 21, Der f 1, Der f 2, Der f 3, Der f 6, Der f 7, Der f 10, Der f 11, Der f 13, Der, f 14, Der f 15, Der f 16, Der f 17, Der f 18, Der f 22, Der m 1, Der p 1, Der p 2, Der p 3, Der p 4, Der p 5, Der p 6, Der p 7, Der p 8, Der p 9, Der p 10, Der p 11, Der p 14, Der p 20, Der p 21, Der p 23, Eur m 1, Eur m 2, Eur m 3, Eur m 4, Eur m 14, Bla g 1, Bla g 2, Bla g 4, Bla g 5, Bla g 6, Bla g 7, Bla g 8, Per a 1, Per a 3, Per a 6, Per a 7, Per a 9 and Per a 10;
- Can f 1, Can f 2, Can f 3, Can f 4, Can f 5, Can f 6, Fel d 1 ,Fel d 2, Fel d 3, Fel d 4, Fel d 5w, Fel d 6w, Fel d 7, Fel d 8, Equ c 1, Equ c 2, Equ c 3, Equ c 4 and Equ c 5;
- Cla c 9m Cla c 14, Cla h 2, Cla h 5, Cla h 6, Cla h 7, Cla h 8, Cla h 9, Cla h 10, Cla h 12. Asp fl 13, Asp f 1, Asp f 2, Asp f 3, Asp f 4, Asp f 5, Asp f 6, Asp f 7, Asp f 8, Asp f 9, Asp f 10, Asp f 11, Asp f 12, Asp f 13, Asp f 15, Asp f 16, Asp f 17, Asp f 18, Asp f 22, Asp f 23, Asp f 27, Asp f 28, Asp f 29, Asp f 34, Asp n 14, Asp n 18, Asp n 25; Asp o 13, Asp o 21, Pen b 13, Pen b 26, Pen ch 13, Pen ch 18, Pen ch 20, Pen ch 31, Pen ch 33, Pen ch 35, Pen c 3, Pen c 13, Pen c 19, Pen c 22, Pen c 24; Pen c 30, Pen c 32, Pen o 18, Fus c 1, Fus c 2, Alt a 1, Alt a 3, Alt a 4, Alt a 5, Alt a 6, Alt a 7, Alt a 8, Alt a 10, Alt a 12, Alt a 13, Cand a 1, Cand a 3 and Cand b 2;

- Cha o 1, Cha o 2, Cup a 1, Cup s 1, Cup s 3, Jun a 1, Jun a 2, Jun a 3, Jun o 4, Jun s 1, Jun v 1 and Jun v 3, Ant o 1, Cyn d 1, Cyn d 7, Cyn d 12, Cyn d 15, Cyn d 22w, Cyn d 23, Cyn d 24, Dac g 1, Dac g 2, Dac g 3, Dac g 4, Dac g 5, Fes p 4, Hol l 1, Hol l 5, Hor v 1, Hor v 5, Lol p 1, Lol p 2, Lol p 3, Lol p 4, Lol p 5, Lol p 11, Pas n 1, Pha a 1, Pha a 5, Phl p 1, Phl p 2, Phl p 4, Phl p 5, Phl p 6, Phl p 7, Phl p 11, Phl p 12, Phl p 13, Poa p 1, Poa p 5, Sec c 1, Sec c 5, Sec c 20, Sor h 1, Tri a 15, Tri a 21, Tri a 27, Tri a 28, Tri a 29, Tri a 30, Tri a 31, Tri a 32, Tri a 33, Tri a 34, Tri a 35, Zea m 1 Zea m 12, Amb a 1, Amb a 2, Amb a 3, Amb a 4, Amb a 5, Amb a 6, Amb a 7, Amb a 8, Amb a 9 and Amb a 10; Amb p 5; Amb t 5; Art v 1, Art v 2, Art v 3, Art v 4, Art v 5, Art v 6, Aln g 1, Aln g 4, Bet v 1, Bet v 2, Bet v 3, Bet v 4, Bet v 6, Bet v 7, Cor a 10, Fra e 1, Ole e 1, Ole e 2, Ole e 3, Ole e 4, Ole e 5, Ole e 6, Ole e 7, Ole e 8, Ole e 9, Ole e 10, Ole e 11, Pla a 1, Pla a 2, Pla a 3, Pla or 1, Pla or 2 and Pla or 3;
- Api c 1, Api d 1; Api m 1, Api m 2, Api m 3, Api m 4, Api m 5, Api m 6, Api m 7, Api m 8, Api m 9, Api m 10, Api m 11, Bom p 1, Bom p 4; Bom t 1, Bom t 4, Dol a 5, Dol m 1, Dol m 2, Dol m 5, Pol a 1, Pol a 2 and Pol a 5, Pol d 1, Pol d 4, Pol d 5, Pol e 1, Pol e 4 and Pol e 5, Pol f 5; Pol g 1, Pol g 5, Poly p 1, Poly s 5; Vesp c 1, Vesp c 5, Vesp ma 2, Vesp ma 5, Vesp m 1, Vesp m 5, Ves g 5, Ves m 1, Ves m 2, Ves m 5; Ves p 5; Ves s 1, Ves s 5; Ves vi 5; Ves v 1, Ves v 2, Ves v 3 and Ves v 5:
- Hev b 1, Hev b 2, Hev b 3, Hev b 4, Hev b, 5, Hev b 6, Hev b 7, Hev b 8, Hev b 9, Hev b 10, Hev b 11, Hev b 12, Hev b 13 and Hev b 14;
- Hor v 12, Hor v 15, Hor v 16, Hor v 17, Hor v 21, Ory s 12, Sec c 20, Tri a 12, Tri a 14, Tri a 18, Tri a 19, Tri a 21, tri a 25, Tri a 26, Tri a 36, Zea m 14, Zea m 25, Ara h 1, Ara h 2, Ara h 3, Ara h 4, Ara h 5, Ara h 6, Ara h 7, Ara h 8, Ara h 9, Ara h 10, Ara h 11, Cor a 1, Cor a 2, Cor a 8, Cor a 9, Cor a 11, Cor a 12, Cor a 13, Cor a 14, Jug n 1, Jug n 2, Jug r 1, Jug r 2, Jug r 3, Jug r 4, Pru du 3, Pru du 4, Pru du 5, Pru du 6, Ana o 1, Ana o 2, Ana o 3, Pis v 1, Pis v 2, Pis v 3, Pis v 4, Pis v 5, Gly m 1, Gly m 2, Gly m 3, Gly m 4, Gly m 5 and Gly m 6; and/or
- casein of cow's milk, beta-lacto-globulin of cow's milk, ovalbumin of chicken egg white, ovomucoid of chicken egg white andallergen M of a fish.

It might be summarized that typical examples of environmental source materials originating from pollen are, but not limited to
- plant and tree pollen of various species of the genus Ambrosia (ragweeds); of the genus Alder (Alnus); of the genus Artemisia; of the genus Betula (Birch); of the genus Dactylis (Orchard Grass); of the genus Quercus (Oaks); of the genus Olea (Olive Oil Trees); of the genus Parietaria (weeds); of the genus Cupressus (Redwoods)); of the genus Juniperus (Cedar trees); Festuca (Fescue grasses); of the genus Avena; of the genus Holcus; of the genus Anthoxanthum; of the genus Arrhenatherum; of the genus Agrostis; of the genus Phleum (Timothy grasses); of the genus Poa (Blue Grass); of the genus Cynodon (Bermuda Grass) of the genus Phalaris; of the genus Paspalum; of the genus Lolium (Rye grass).

Typical examples of environmental source materials of animal origin are, but not limited to;
- dander, hair, urine and faeces of the genus canis, of the family felis, of the genus Equus, of the genus Periplanbeta (American cockroach), of the genus Blomia (storage mite) and of the genus Dermatophagoides;

Typical examples of environmental source materials of fungal spores, yeast and moulds are, but not limited to - fungal spores, yeast and moulds of various species from the genus Alternaria, Aspergillus and Cladosporium;

Still other examples of environmental source material are venoms derived from insects, such as venoms from the class of insects named hymenoptera, more specifically from the insects of the family named Apidae (such as honey bees and bumble bees) and the family named Vespidae (such as wasps (the Vespula species), hornets, and paper wasps).

In particular interesting embodiments, the environmental source material is pollen of various species of the genus Ambrosia (ragweeds); of the genus Alder (Alnus); of the genus Betula (Birch); of the genus Olea (Olive Oil Tree); of the genus Cupressus (Redwoods); of the genus Juniperus (Cedar) and of the genus Phleum.

As mentioned, a source material of the invention may also be a dietary inclusive foods, feeds, drinks and industrial produced dietary's. Typical examples, on such source materials are, but not limited to, various species of nuts, such as of the tree nuts including macadamia nuts, brazil nuts, cashews, almonds, walnuts, pecans, pistachios, chestnuts, beechnuts, hazelnuts, pine nuts, gingko nuts and hickory nuts as well as industrial dietary products therof.

Other food sources are cereals containing gluten (i.e. wheat, rye, barley, oats, spelt); fish; crustaceans; egg; peanut; soybeans (soy protein, textured vegetable protein TPV, hydrolysed plant protein, hydrolysed soy protein, hydrolysed vegetable protein); milk and dairy products including lactose (milk sugar); nuts (e.g. almond (Amygdalus communis), hazelnut (Corylus avellana), walnut (Juglans regia), cashew (Anacardium occidentale), pecan nut(Carya illinoiesis), Brazil nut (Bertholletia excelsa), pistachio nut (Pistacia vera), macadamia nut, Queensland nut (Macadamia ternifolia)); celery and other foods of the Umbelliferae family; mustard; and sesame seed as well as industrial dietary products thereof.

### Treatment and administration of unrelated antigen to oral cavity

As mentioned, the antigen of the invention is used in treatment of a hypersensitivity immune response. More particularly, the hypersensitivity immune response is associated with an allergic disease/allergic immune response, such as a type 1 or a type 4 hypersensitivity immune response.

Typical examples of diseases mediated by a hypersensitivity immune response are, but not limited to, allergic diseases like atopic dermatitis, urticaria, contact dermatitis, allergic conjunctivitis, allergic rhinitis, allergic asthma, anapylaxis, food allergy, drug allergy.

Typical examples of diseases mediated by a type 1 hypersensitivity immune reaction are, but not limited to, allergic diseases like atopic dermatitis, allergic conjunctivitis, allergic rhinitis, allergic asthma, anapylaxis, food allergy, drug allergy and hay fever.

In particularly, a hypersensitivity immune response of the respiratory tract is associated to allergic rhinitis and/or allergic asthma; a hypersensitivity immune response of the gastro-intestinal tract is associated to food allergy and a hypersensitivity immune response of the skin is associated to contact dermatitis and/or atopic dermatitis.

It is anticipated that the administration to mucosa of the oral cavity may be performed by topically delivering the antigen at the surface of an epithelia of the oral cavity from where the antigen is absorbed into the mucosa and sub mucosa. For example, the antigen is administered to the epithelia of the sublingual part including the floor of the mouth from where the antigen is absorbed into the mucosa and sub mucosa. It should be understood that in more interesting embodiments of the invention, the antigen is administered by sublingual administration.

Therefore, in still further embodiments of the invention, the antigen of the invention is administered by sublingual administration, i.e. topically to the sublingual epithelia or directly to the sublingual mucosa. As the sublingual route forms part of the oral cavity, it is also preferred to administer the unrelated antigen to the epithelia or mucosa of the oral cavity, such as by buccal administration. By buccal administration it is the intention to apply a suitable pharmaceutical formulation of the unrelated antigen on a mucosa of the oral cavity, such as the sublingual mucosa, at the upper surface of the tongue, at the gum or at the cheeks, so as to deliver the unrelated antigen to the mucosa of the oral cavity and wherein it is not the intention to deliver the unrelated antigen to the gastro-intestinal tract. Thus, in more preferable embodiments of the invention, the antigen is administered to the mucosa of the oral cavity, such as by sublingual administration, but where the individual is instructed not to swallow the unrelated antigen before a period of at least 1 minute after the administration of the unrelated antigen, such as more preferably, after 2 minutes, 3 minutes, 4 minutes, or 5 minutes of administration of the unrelated antigen.

As mentioned, the unrelated antigen of the invention might be used in the treatment of a hypersensitivity immune reaction. The dosage regimen of relevance might be one usually applied in the field of allergen specific immunotherapy, for example, in terms of selecting doses, number of doses per day, duration of treatment and frequency of administration. Thus, it might be envisaged that the unrelated antigen be administered frequently during a longer period before the desirable effect is achieved, such as administered daily to the mucosa of the oral cavity (e.g. sublingual mucosa) for a period of at least 6 months. Also it is envisaged that where the hypersensitivity immune response is caused by a seasonal allergen, the first dose may be administered before the allergen season.

It is also envisaged that the treatment is initiated by an up-dosing phase where the unrelated antigen is administered in increasingly doses during one day or with days between until a maintenance dose is achieved.

It should be understood that the unrelated antigen is the only antigen administered in the present treatment regiment to the oral cavity. In particularly, the allergen to which the individual has specific IgE antibodies towards shall not be co-administered with the unrelated antigen. Most suitable an allergen is not co-administered with the unrelated antigen at all. It should also be understood that the unrelated antigen is not administered together with a modulator of the Notch signalling pathway either as a combined preparation for simultaneous, contemporaneous, separate or sequential use for modulation of the immune system as described in WO 2004/082710.

### Co-exposure/Co-availability

As mentioned, it is crucial to the invention, that the individual be co-exposed to the unrelated antigen, at least at the time the individual is exposed to an allergen-containing source material comprising the allergen triggering the hypersensitivity immune response in the individual.

Accordingly, the unrelated antigen is also administered to the target organ, such as the respiratory tract, gastro-intestinal tract, or skin within a period at least coinciding partly or entirely with the individual's exposure to said source material.

The phrase "the antigen is also administered ....within a period at least coinciding partly or entirely with the individual's exposure to a source material" is meant to designate that the unrelated antigen is administered to the target organ that is exposed to the "triggering" allergen and that the administration of the unrelated antigen shall be performed at least during the entire period of allergen exposure or it might be administered in a part of that period.

Therefore, in various embodiments of the invention, the unrelated antigen is administered to an individual in need thereof by combined administration of the unrelated antigen to the mucosa of the oral cavity and the administration of the same unrelated antigen or a variant thereof performed simultaneously, contemporaneously, separately or sequentially, in either order.

It is not considered that the unrelated antigen need not be administered to the target organ simultaneous and within the same period of time where the target organ is exposed to the allergen, e.g. to the respiratory tract, gastro-intestinal tract, or to the skin, as the time period may be adjusted to as to achieve bystander suppression. That is to say that the phrase "in a period coinciding with the exposure to" is meant to encompass that the unrelated antigen is administered just before exposure, at the start of exposure, at the end of exposure, within the entire period of exposure, almost within in the period of exposure, and now and then during the exposure. For example, the unrelated antigen is presented to the target organ few hours (1-6 hours), few days (1-4 days) or few (1-5 weeks) or few months (1-5 months) before the allerergen exposure takes place at the target organ. However, preferably, the unrelated antigen is administered to, exposed to or available at the target organ substantially within in the same period as the "triggering" allergen, such as at least simultaneously.

For example, where the allergen-containing source material is an airborne environmental source material, e.g. pollen, mite feces, mite crops, animal dander, animal hair, animal stool, that reaches the respiratory tract, the unrelated antigen might additionally be administered to the respiratory tract, such as to the nasal cavity. For example, a dosing regiment may include the administration of the unrelated antigen to the mucosa of the oral cavity, such as in the form of a sublingual tablet, and the additional administration of the unrelated antigen to the nasal cavity, such as in the form of a nasal spray or nasal drops.

Furthermore, where the allergen-containing source material is a dietary source material, e.g. food or a food-containing product, the unrelated antigen might additionally be administered to the gastro-intestinal tract, such as by ingestion or per-oral administration of a tablet or a capsule.

Likewise, where the allergen-containing source material is an environmental source material, e.g. an environmental source material associated with allergic responses of the skin (e.g. a gum or a gum-containing product), the unrelated antigen might additionally be administered to the skin, such as in the form of a crème, paste, gel or plaster.

It should also be understood that the additional administration of the unrelated antigen to the respiratory tract, gastro-intestinal tract, or skin need not be limited to the period of exposure to the allergen, but may additionally take place out-site the period of exposure to the allergen triggering the hypersensitivity immune response.

It is anticipated that in such embodiments, where the unrelated antigen is administered to the mucosa of the oral cavity as well as to either; the respiratory tract, gastro-intestinal tract or skin, this might be performed simultaneously, contemporaneously, separately or by sequentially administering, in either order: i) a therapeutically effective amount of an unrelated antigen to the oral cavity; and ii) an effective amount of the same unrelated antigen or an variant thereof to either the gastro-intestinal tract, gastro-intestinal tract of skin. At least it might be envisaged that the first step in treatment or prophylactic treatment ofis the administration of the unrelated antigen to the mucosa of the oral cavity. Furthermore, the additional administration of the unrelated antigen to the respiratory tract, gastro-intestinal tract or skin is initiated subsequently, such as days, months or years after starting the administration of unrelated antigen to the oral mucosa. Thus, in some embodiments of the invention, the administration of the unrelated antigen to the oral cavity is initiated before the administration of the unrelated antigen to the respiratory tract, gastro-intestinal tract or skin.

It is also envisaged that the additional administration of the unrelated antigen at least be performed during the individual's exposure to the "triggering" allergen, such as during the pollen season or when ingesting allergen-containing foods. It is also envisaged that the additional administration of the unrelated antigen be continued after the administration of the unrelated antigen to the mucosa of the oral cavity has stopped. Thus, in various embodiments of the invention, the administration of the unrelated antigen to the respiratory tract, gastro-intestinal tract, or skin at least be performed during the individual's exposure to an allergen-containing source material of interest, such as to an allergen to which the individual is sensitized to, such as to an allergen triggering a hypersensitivity immune response in the individual or in a group of individuals of interest; the administration of the unrelated antigen to the respiratory tract, gastro-intestinal tract, or skin is initiated in the period of administering the unrelated antigen to the oral cavity and continued after the administration to the oral cavity has stopped; the administration of the unrelated antigen to the oral cavity is performed daily, twice weekly, weekly or twice a month; and/or the administration of the unrelated antigen to the respiratory tract, gastro-intestinal tract, or skin is performed daily, twice weekly, weekly or twice a month.

In interesting embodiments of the invention, the unrelated antigen is administered to the oral cavity, such as by sublingual administration, such as by sublingual administration of a tablet (including fast-disintegrating tablet, freeze-dried tablet) and the same unrelated antigen or a variant thereof is administered to the nasal cavity in the form of a nasal spray or nasal drops.

*Slightly differences between unrelated antigen for administration to oral cavity versus administration to the respiratory tract, gastro-intestinal tract or skin.* It should also be considered that the unrelated antigen as administered to the respiratory tract, gastro-intestinal tract or skin need not be identical to unrelated antigen administered to the oral cavity. However, still preferably, the unrelated antigen used in the step of administering the unrelated antigen to the oral cavity is the same as the unrelated antigen used in the administration of unrelated antigen to the respiratory tract (e.g. nasal cavity), gastro-intestinal tract (e.g. per-oral administration or by ingestion) or to the skin.

However, it is considered that the variant of the unrelated antigen is chemical modified or biologically modified so as to modify its solubility properties, bioavailability properties or stability. Examples are antigen variants with chemical modifications, such as N- or O-linked glycosylation or derivatizing of the N-terminus or of thiol groups of the unrelated antigen. Other examples are antigen variants that are biologically modified, such as by post-translational modifications, such as for example; glycosylation, acetylation, alkylation (methylation, ethylation), biotinylation, glutamylation, glycylation, isoprenylation, lipoylation, phosphopantetheinylation, phosphorylation, sulfation, selenation and C-terminal amidation.

### Formulations

Where the antigen exhibits poor stability to the gastric juice, the unrelated antigen is preferably administered in a form avoiding the contact to the gastric juice, such as in a form preventing the degradation of the unrelated antigen in the gastric juice. This may be accomplished by incorporating the unrelated antigen in pharmaceutical formulations resistant to the gastric juice or by incorporating other pharmaceutical delivery techniques able to avoiding the degradation of proteins in the gastric fluid.

The antigen of the invention may be formulated together with therapeutically inactive ingredients and/or immune-modifying agents like adjuvants. Typically, the formulation is a solid dosage form, such as a fast-disintegrating tablet or a liquid including a solution, a suspension, a dispersion, a gelled liquid. Alternatively, the formulation is an emulsion or a re-dissolvable powder, granulate or lyophilisate, which can be dissolved to form a liquid before being administered.

Excipients for use in formulations are well-known to the person skilled in the art and include solvents, emulsifiers, wetting agents, plasticizers, colouring substances, fillers, preservatives, viscosity adjusting agents, buffering agents, pH adjusting agents, isotonicity adjusting agents, mucoadhesive substances, and the like. Examples of formulation strategies are well-known to the person skilled in the art.

The adjuvant may be any conventional adjuvant, including oxygen-containing metal salts, e.g. aluminium hydroxide, chitosan, heat-labile enterotoxin (LT), cholera toxin (CT), cholera toxin B subunit (CTB), polymerised liposomes, mutant toxins, e.g. LTK63 and LTR72, microcapsules, interleukins (e.g. IL-1 BETA, IL-2, IL-7, IL-12, INFGAMMA), GM-CSF, MDF derivatives, CpG oligonucleotides, LPS, MPL, phosphophazenes, Adju-Phos(R), glucan, antigen formulation, liposomes, DDE, DHEA, DMPC, DMPG, DOC/Alum Complex, Freund's incomplete adjuvant, ISCOMs(R), LT Oral Adjuvant, muramyl dipeptide, monophosphoryl lipid A, muramyl thpeptide, and phospatidylethanolamine.

### List of References

Brimnes et al. Sublingual immunotherapy reduces allergic symptoms in a mouse model of rhinitis. Clinical and experimental allergy, vol 37, no 4, p 488-497, 2007.
Brimnes et al. Sublingual immunotherapy (SLIT) induce systemic tolerance to naïve mice. J Clin Allergy and Clinical Immunology, vol 123, 2, p S127, 2009. Dahlmann-Hoglund et al. Bystander suppression of the immune response to human serum albumin in rats, Immunology 86, 128-133, 1995.
Dunkin D, Berin MC, Mayer L. Allergic sensitization can be induced via multiple physiologic routes in an adjuvant-dependent manner. J Allergy Clin Immunol 2011.
Jin H, He R, Oyoshi M, Geha RS. Animal models of atopic dermatitis. J Invest Dermatol 2009; 129(1):31-40.
Kildsgaard et al. Sublingual immunotherapy (SLIT) in sensitized mice induces mucosal IgA antibodies. J Clin Allergy and Clinical Immunology, vol 115, 2, p S207, 2005.
Kildsgaard et al. Sublingual immunotherapy in sensitized mice. Annals of allergy, vol 98, 4, p 366-372, 2007.
Miller et al. Antigen-driven Bystander Suppression after Oral Administration of Antigens, J. Exp. Med. 174, 791-798, 1991.
Millington et al. Induction of Bystander Suppression by Feeding Antigen Occurs despite Normal Clonal Expansion of the Bystander T Cell Population. Immunology, 173: 6059-6064, 2004.
Oliveira CR et al. Bystander effect in synergi to anergy in oral tolerance of Blomia Tropcalis/Ovalbumin Murine Co-Immunization model. J Clin Immunol, 25, 153-161, 2005.
Ramos et al. Cell-mediated immune response to unrelated proteins and unspecific inflammation blocked by orally tolerated proteins. Immunology, 126, 354-362, 2008.
Rask et al. Shorter dosing intervals of sublingual immunotherapy leads to more efficacious treatment in a mouse model of allergic inflammation. Scandinavian journal of immunology, vol 71, no 6, p 403-412, 2010.
Sabatos-Peyton C et al. Antigen-specific immunotherapy of autoimmune and allergic diseases. Current opinion in Immunology, 22, 1-7, 2010.
Spergel JM, Mizoguchi E, Oettgen H, Bhan AK, Geha RS. Roles of TH1 and TH2 cytokines in a murine model of allergic dermatitis. J Clin Invest 1999; 103(8):1103-11.
Wang Lifang et al. Antigen driven bystander effect accelerates epicutaneous sensitization with a new protein allergen. J Biomed Science, vol 16, 28, p 1423-0127, 2009.
Weiner et al. Oral tolerance; immune mechanisms and treatment of autoimmune diseases. Immunology of today, vol 18, no 7, p 335-343, 1997.

### Examples

### Example 1

**Prophylactic treatment of naive mice with an unrelated antigen.**

### Methods:

An extract of grass pollen of the species phleum pratense (Phl p) is obtained by extracting defatted grass pollen with an aqueous saline solution.

### Animals

Female, 6-10 week-old BALB/cJ mice were bred in-house and maintained on a defined diet not containing component cross reacting with antisera to Phl p. Each experimental group consisted of 8 animals.

### Animal experiments

Naïve mice received sublingual immunotherapy (SLIT) with 40 µg Phl p extract or buffer for two weeks before being immunized to raise an immune response against another antigen. SLIT was performed by holding the scruff of the mice and carefully applying 5 µl of allergen solution under the tongue. The mice were held by the scruff for additional 20 seconds to prevent the animal from swallowing the allergen solution. The mice were then challenged by intraperitoneal injection of either 8 µg Phl p extract mixed with 250 µg chicken ovalbumin (OVA) adsorbed to aluminium hydroxide or of OVA alone adsorbed to aluminium hydroxide so as to develop an immune response. Ten to twelve days later the mice were euthanized, spleens were removed and cells were set up in an in vitro re-stimulation assay. In this assay, cells were stimulated with the antigen triggering an immune response (OVA) in order to evaluate the effect of SLIT treatment of mice with the unrelated antigens present in the Phl p extract on the development of an OVA specific immune response. In alternative experiments mice were SLIT treated with OVA and intraperitoneally injected with Phl p extract mixed with OVA or Phl p extract alone. Following euthanization, spleen cells were re-stimulated by Phi p to evaluate the effect of the sublingual pre-treatment with OVA on the Phl p specific immune response. The experimental outline is depicted in figure 1.

### T-cell proliferation assay

Spleens were teased into single cell suspension and washed three times in medium. Cells were counted and adjusted to 1.67 x 106 cells/mL. 3 x 10⁵ cells were added to each well of a 96 well flat-bottomed culture plate and the cells were stimulated by 0, 5, 25 and 125 µg/mL OVA. The cells were cultured for 6 days at 37 °C and 5% CO₂. Proliferation was measured by adding 0.5 µCi of 3H-thymidine to each well for the last 18 hours of the culture period, followed by harvesting the cells and counting the incorporated radiolabel.

### Cytokine measurements

Spleens were teased into single cell suspension and washed three times in medium. Cells were counted and adjusted to 3 x10⁶ cells/mL. 5 x 10⁶ cells were added to each well of a 96 well culture plate and the cells were stimulated by 0 and 125 µg/mL OVA. Supernatants were harvested at day 5 and analyzed for the presence of the cytokines IL-4 and IL-5 using a Mesoscale mouse Th1/Th2 ELISA kit.

### Results

### SLIT treatment with Phl p

Figure 2 shows the proliferation rate of spleen cells from mice that have either been SLIT pre-treated with Phl p or with buffer. Following the SLIT treatment, the mice were i.p. immunized with either OVA plus Phl p together (panel A) or OVA alone (panel B) and subsequently spleen cells were re-stimulated in vitro with OVA. The results of Figure 2 indicate that prophylactic SLIT treatment with an antigen (Phl p) unrelated to the antigen triggering the immune response (OVA) down regulates the splenic T-cell response against OVA. However this only occurs when Phl p is present together with OVA at the time of immunization. Also as shown in figure 3, the SLIT pre-treatment with Phl p is capable of down regulating the levels of the cytokines IL-4 and IL-5 in the OVA stimulated T-cell cultures. The cytokines IL-4 and IL-5 are induced by the i.p. immunization with OVA antigen and are cytokines usually induced in Th2-driven immune response.

### SLIT treatment with OVA

Figure 4 shows the proliferation of spleen cells from mice that have been SLIT treated with either OVA or buffer. Following the SLIT treatment the mice were i.p. immunized with either Phl p and OVA together (panel A) or Phl p alone (panel B) and subsequently spleen cells were re-stimulated in vitro with Phl p. The results indicate that prophylactic SLIT treatment with OVA (unrelated antigen) is able to down regulate the splenic T-cell response against Phl p.

Together these data demonstrate that suppression of an immune response (characterized by Th2-cytokines) by SLIT treatment of the mice with an antigen unrelated to the antigen triggering the immune response can be obtained, regardless whether OVA or Phl p extract served as model antigens of the unrelated antigen.

### Example 2

### Comparison of the sublingual route versus the per-oral route of administering an unrelated antigen

### Methods:

Phl p, an extract of grass pollen of the species phleum pratense is obtained by extracting defatted grass pollen with an aqueous saline solution.

### Animals

Female, 6-10 week-old BALB/cJ mice were bred in-house and maintained on a defined diet not containing component cross reacting with antisera to Phl p. Each experimental group consisted of 8 animals.

### Animal experiments

Naïve mice were treated daily with 40 µg Phl p extract either by the sublingual or the peroral (intragastric gavache) route for two weeks. The mice were then immunized to raise an immune response against another antigen by intraperitoneal injection. This injection consisted of 8 µg Phl p extract mixed with 250 µg ovalbumin (OVA) adsorbed to aluminium hydroxide. Ten to twelve days later the mice were euthanized, spleens were removed and cells were set up in an in vitro re-stimulation assay. In this assay the cells were stimulated with OVA in order to evaluate the effect of SLIT treatment of with the unrelated antigen Phl p on the OVA specific immune response. The experimental outline is depicted in figure 5.

### T-cell proliferation assay

Spleens were teased into single cell suspension and washed three times in medium. Cells were counted and adjusted to 1.67 x 10⁶ cells/mL. 3 x 10⁵ cells were added to each well of a 96 well flat-bottomed culture plate and the cells were stimulated by 0, 5, 25 and 125 µg/mL OVA. The cells were cultured for 6 days at 37°C and 5% CO2. Proliferation was measured by adding 0.5 µCi of 3H-thymidine to each well for the last 18 hours of the culture period, followed by harvesting the cells and counting the incorporated radiolabel.

### Results:

As seen in figure 6, the OVA-specific proliferation of spleen cells from mice SLIT treated with Phl p prior to the i.p. immunization with Phl p and OVA together was significantly decreased compared to buffer treated mice. This was the case regardless whether the spleen cells were stimulated with a high or low dose of OVA. In contrast, the proliferation of spleen cells from mice per-orally treated with the same dose of Phl p as the SLIT treated mice, was not down regulates to the same extent as observed in the SLIT treated mice. These data shows that SLIT is more effective in suppression an immune response triggered by another antigen compared to per-oral treatment.

### Example 3

### Prophylactic treatment of naive mice with an unrelated antigen to reduce clinical relevant symptoms on a hypersensitivity immune response

### Methods:

Phl p, an extract of grass pollen of the species phleum pratense is obtained by extracting defatted grass pollen with an aqueous saline solution.

### Animals

Female, 6-10 week-old BALB/cJ mice were bred in-house and maintained on a defined diet not containing component cross reacting with antisera to Phleum pratense (Phl p). Each experimental group consisted of 8 animals.

### Animal experiments

Naive mice were treated by sublingual immunotherapy (SLIT) with 40 µg Phl p extract for 2 weeks. Subsequently, the mice were immunized by three weekly i.p. injections of either a mix of 10 µg OVA and 8 µg Phl p extract or 10 µg OVA alone adsorbed to aluminium hydroxide. Subsequently, the mice were challenged intra-nasally (IN) with 50 µg of OVA for four days so as to induce clinically relevant readouts of a Th2-driven immune response. The mice were sacrificed one day after the last challenge and blood, bronchoalveolar fluid (BAL), spleen and cervical lymph nodes were collected for analysis. The experimental outline is depicted in figure 7.

### Evaluating clinically relevant readouts

### Clinically relevant readouts, such as airway hyper-reactivity and the fraction of eosinophils, were obtained on the last day of IN challenge.

*Airway hyper-reactivity:* Using a whole body pletysmograph, airflow obstruction was induced by challenging the mice with increasing concentrations of aerosolized metacholine. Pulmonary airflow obstruction was measured by enhanced pause (penh) in a period of 6 minutes after administration of metacholine.

*Differential counting of BAL fluid:* The BAL fluid was centrifuged and the supernatant was removed. The pellet was re-suspended in PBS and the fraction of eosinophils was determined by an automated cell counter (Sysmex).

### T-cell proliferation assay

Spleens were teased into single cell suspension and washed three times in medium. Cells were counted and adjusted to 1.67 x 10⁶ cells/mL. 3 x 10⁵ cells were added to each well of a 96 well flat-bottomed culture plate and the cells were stimulated by 0, 5, 25 and 125 µg/mL OVA. The cells were cultured for 6 days at 37 °C and 5% CO2. Proliferation was measured by adding 0.5 µCi of 3H-thymidine to each well for the last 18 hours of the culture period, followed by harvesting the cells and counting the incorporated radiolabel.

### Results

By these experiments, the inventors wanted to examine whether the prophylactic SLIT treatment also had an effect on allergic asthma. In short, naïve mice were SLIT treated with an unrelated antigen (Phl p) or with buffer prior to the induction of allergic asthma. The asthma was induced by first immunizing the mice by i.p. injection with OVA (antigen triggering the immune response) and Phl p together versus OVA alone, followed by intranasal challenge with the same OVA. As demonstrated by the results depicted in Figure 8, Phl p SLIT treated mice showed reduced airway hyperresponsiveness compared to buffer treated mice (Panel A), whereas this suppression could not be observed in mice that were only immunized with OVA (panel B). Panel C and D shows the percentage of eosinophils in bronchoalveolar lavage (BAL) fluid. Here it can be seen that the fraction of eosinophils was down regulated in mice SLIT treated with an unrelated antigen compared to mice SLIT treated with buffer. Again this suppression could only be observed, when the unrelated antigen (Phl p) was co-administered together with the sensitizing antigen (OVA) at the i.p. immunization. Panels E and F show in-vitro proliferation of cervical lymph node cells. In line with the ability to down-regulate eosinophils in BAL, the OVA-specific in vitro proliferation of cLN cells was down regulated in the Phl p SLIT treated mice, but only when both the sensitizing antigen and the unrelated antigen was present at the time during the i.p. injection.

The data in this series of experiments show that prophylactic SLIT treatment with an unrelated antigen suppresses clinically relevant readouts of allergic asthma in mice.

### Example 4

### Treatment of sensitized mice with an unrelated antigen to reduce clinical relevant symptoms on a hypersensitivity immune response

### Methods:

Phl p, an extract of grass pollen of the species phleum pratense is obtained by extracting defatted grass pollen with an aqueous saline solution.

### Animals

Female, 6-10 week-old BALB/cJ mice were bred in-house and maintained on a defined diet not containing component cross reacting with antisera to Phleum pratense (Phl p). Each experimental group consisted of 8 animals.

### Animal experiments

In one set of experiments mice were sensitized by three weekly i.p. injections of 8 µg Phl p extract adsorbed to aluminium hydroxide. Subsequently, the mice were treated by sublingual immunotherapy (SLIT) with 250 µg OVA for 4 weeks, followed by 2 weeks of intranasal challenge with 8 µg Phl p together with 10 µg OVA or Phl p alone. The experimental outline is depicted in figure 9.

In another set of experiments, mice were sensitized by two weekly i.p. injections of 10 µg OVA adsorbed to aluminium hydroxide. Following this, the mice were SLIT treated with 40 µg Phl p for 4 weeks and subsequently they were intra-nasally challenged for 4 days with 100 µg OVA together with 8 µg Phl p or with 8 µg OVA alone. The experimental outline of this is depicted in figure 13.

In both sets of experiments, the mice were sacrificed one day after the last challenge and blood, bronchoalveolar fluid (BAL), spleen and cervical lymph nodes were collected for analysis.

### Evaluating clinically relevant readouts

Clinically relevant readouts, such as sneezes, airway hyper-reactivity and presence of eosinophils, were obtained on the last day of IN challenge.

*Sneezing:* The mice were observed in an 8 min-period after intranasal administration of Phl p and the numbers of sneezes were counted during this period.

*Airway hyper-reactivity:* Using a whole body pletysmograph, airflow obstruction was induced by increasing concentrations of aerosolized metacholine. Pulmonary airflow obstruction was measured by enhanced pause (penh) in a period of 6 minutes after administration of metacholine.

*Differential counting of BAL fluid:* The BAL fluid was centrifuged and the supernatant was removed. The pellet was re-suspended in PBS and the fraction of eosinophils was determined by an automated cell counter (Sysmex).

### Results

### Suppression induced by SLIT treatment with Phl p

In this set of experiments the inventors wanted to investigate if SLIT treatment with one antigen (Phl p) could prevent clinical signs of asthma induced by another antigen (OVA) in a mouse animal model of asthma. As shown in Figure 10 Panel A, the fraction of eosinophils in the BAL fluid was significantly decreased in OVA sensitized mice SLIT treated with Phl p (unrelated antigen) versus mice SLIT treated with buffer, upon the subsequent intranasal challenge with OVA (antigen triggering the immune response) and Phl p together. This down regulation of eosinophils could not be observed if the mice were intra-nasally challenged with OVA alone (Figure 10, Panel B). Furthermore, as seen in figure 11A, the SLIT treatment with Phl p suppressed the IL-5 levels in the BAL fluid compared to SLIT buffer treated mice, provided that the mice were exposed to both OVA and Phl p upon intranasal challenge. This downregulation could not be observed in mice that were IN challenged with OVA alone (Fig 11B). Finally, Figure 12 demonstrates a tendency to the suppression of the airway hyper responsiveness in the Phl p SLIT treated mice. Again, this effect was dependent on the co-exposure of OVA and Phl p during the intranasal challenge.

### Suppression induced by SLIT treatment with OVA

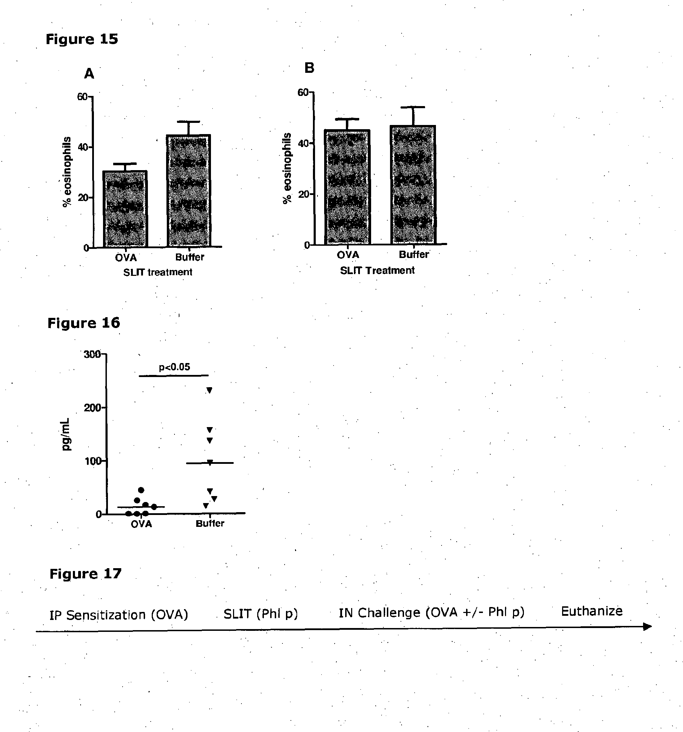

In this set of experiments, the inventors investigated if SLIT treatment with an unrelated antigen (OVA) could prevent clinical and immunological signs of asthma induced by another antigen (phl p) in a mouse animal model of rhinitis. In the rhinitis model employed, the primary clinical read-out is the number of sneezes. As shown in Figure 14, Panel A, Phl p sensitized mice SLIT treated with OVA had significantly less sneezes compared to buffer SLIT treated mice upon the intranasal challenge. This reduction in sneezes was dependent on the presence of both the antigen used for SLIT treatment (OVA) as well as the antigen (Phl p) responsible for inducing allergic rhinitis (Figure 14, Panel B). Furthermore, as observed in Figure 15, Panel A, the fraction of eosinophils in the BAL fluid isolated from OVA SLIT treated mice tend to be reduced, but only if both Phl p and OVA (unrelated antigen) were both presence upon intranasal challenge (Figure 15 Panel B). Finally, as seen in Figure 16, the levels of IL-5 in cell culture supernatants of cLN cells obtained from phl p pre-sensitized mice SLIT treated with OVA were also significantly suppressed upon the intra-nasal challenge with both the sensitizing antigen (Phl p) and OVA (unrelated antigen) together.

In summary, these results show that it is possible to suppress a clinically relevant readout of allergic rhinitis in mice pre-sensitized to an antigen by SLIT treatment of the mice with an unrelated antigen provided the mice are exposed to both the antigen triggering the allergic rhinitis and the unrelated antigen upon challenging respiratory organ of the mice to the triggering antigen.

### Example 5

### Treatment of sensitized mice with an unrelated antigen to reduce clinical relevant symptoms on a hypersensitivity immune response

### Methods:

Phl p, an extract of grass pollen of the species phleum pratense is obtained by extracting defatted grass pollen with an aqueous saline solution.

### Animals

Female, 6-10 week-old BALB/cJ mice were bred in-house and maintained on a defined diet not containing component cross reacting with antisera to Phleum pratense (Phl p). Each experimental group consisted of 8 animals.

### Animal experiments

Mice were sensitized by two weekly i.p. injection of 10 µg OVA adsorbed to aluminium hydroxide. Following this, the mice were SLIT treated with 40 µg Phl p for 4 weeks and subsequently they were intra-nasally challenged for 4 days with 50 µg OVA together with 8 µg Phl p or with OVA alone. At the last day of the intranasal challenge, airway hyperresponsiveness was measured using whole body plethysmography. The mice were sacrificed one day after the last challenge and blood, bronchoalveolar fluid (BAL), spleen and cervical lymph nodes were collected for analysis. The experimental outline is depicted in figure 17.

### Evaluating clinically relevant readouts

Clinically relevant readouts, such as airway hyper-reactivity and presence of eosinophils, were obtained on the last day of IN challenge.

*Airway hyper-reactivity:* Using a whole body pletysmograph, airflow obstruction was induced by increasing concentrations of aerosolized metacholine. Pulmonary airflow obstruction was measured by enhanced pause (penh) in a period of 6 minutes after administration of metacholine.

*Differential counting of BAL fluid:* The BAL fluid was centrifuged and the supernatant was removed. The pellet was re-suspended in PBS and the fraction of eosinophils was determined by an automated cell counter (Sysmex).

### Results

In this set of experiments, the inventors wanted to confirm the results of Example 4.

As observed Figure 18, Panel A, the airway hyperresponsesiveness was significantly decreased in OVA pre-sensitized mice subsequently SLIT treated with Phl p (unrelated antigen) compared to mice subsequently SLIT treated with buffer prior to the intranasal challenge with OVA (disease-eliciting antigen) and Phl p together. Moreover, a trend towards a decrease in the fraction of eosinophils in the BAL of Phl p SLIT treated mice could be observed (Figure 18, Panel B). Finally, a decrease in the in vitro spleen cell proliferation was observed in phl p SLIT treated mice upon stimulation with OVA (Figure 18C).

Taken together, these results confirm the results of Example 4, and thereby strengthen the observation that an allergic immune response can be suppressed by treatment of pre-sensitized mice with an unrelated antigen.

### Example 6

### Prophylactic treatment of naïve mice with an unrelated antigen so as to reduce a hypersensitivity immune response.

### Methods:

### Animals

Female, 6-10 week-old BALB/cJ mice were bred in-house and maintained on a defined diet not containing component cross reacting with antisera to Phleum pratense (Phl p). Each experimental group consisted of 8 animals.

### Animal experiments

Naive mice were treated by sublingual immunotherapy (SLIT) with 50 or 200 µg Phl p 5 for 2 weeks, followed by three weekly i.p. injections of either a mix of 10 µg OVA and 10 µg Phl p 5 adsorbed to aluminium hydroxide. Subsequently, the mice were challenged intranasally with 50 µg of OVA for four days. The mice were sacrificed one day after the last challenge and blood, bronchoalveolar fluid (BAL), spleen and cervical lymph nodes were collected for analysis. The experimental outline is depicted in figure 7.

### Clinical data

Clinical data were obtained 24 hours after the last day of IN challenge. *Differential counting of BAL fluid:* The BAL fluid was centrifuged and the supernatant was removed. The pellet was re-suspended in PBS and the fraction of eosinophils was determined by an automated cell counter (Sysmex).

### T-cell proliferation assay

Spleens were teased into single cell suspension and washed three times in medium. Cells were counted and adjusted to 1.67 x 10⁶ cells/mL. 3 x 10⁵ cells were added to each well of a 96 well flat-bottomed culture plate and the cells were stimulated by 0, 5, 25 and 125 µg/mL OVA. The cells were cultured for 6 days at 37 °C and 5% CO2. Proliferation was measured by adding 0.5 µCi of 3H-thymidine to each well for the last 18 hours of the culture period, followed by harvesting the cells and counting the incorporated radiolabel.

### Results:

In this experiment, the results of example 3 are confirmed using a single antigen for the induction of tolerance by the SLIT treatment instead of the Phl p extract used in example 3. This was done by SLIT treatment of naïve mice with Phl p 5 or buffer. After the SLIT treatment allergic asthma was induced by an IP challenge with OVA and Phl p 5 together or OVA alone, followed by intranasal challenge with OVA. As seen in figure 19A, the fraction of eosinophils in the BAL fluid is reduced in mice SLIT treated with Phl p 5 compared to buffer treated mice, when the mice were subsequently sensitized with OVA together with Phl p and intranasally challenged with OVA. Figure 19B shows the spleen cell proliferation upon in vitro re-stimulation with OVA. In this figure it can be seen that SLIT treatment with Phl p 5 is able to downregulate the OVA-specific in vitro proliferation of spleen cells, when Phl p was present at the time of the IP sensitization.

The data in this experiment show that prophylactic SLIT treatment with a single antigen induces bystander tolerance that has an effect on the clinical read-outs of a subsequently induced allergic asthma.

### Example 7

### Detection of unrelated antigen in an allergen source material

### Methods

A number of antigens (unrelated antigen) that do not bind to sera of allergic patients were detected in pollen allergen extracts or mite allergen extracts by crossed radio immunoelectrophoresis (CIE).

Aqueous extracts of pollen allergens of Phleum Pratense (Phl p) or of mite (Dermatophagoides pteronyssinus (Der p) and Dermatophagoides farinae (Der f) allergens (mite bodies and culture medium) were prepared by the following extracting procedure:
For extraction of whole animals (mite bodies), the mite bodies are grinded in a mortar before extraction. Mite culture and pollen are extracted directly.
Extraction 1:10 are carried out as follows; 1 g of pollen (Phl p) or 1 g of mite bodies + mite culture (Der p or Der f) were suspended in 10 ml of aqueous solution of 0.125 M NH₄HCO₃ and 15 mM NaN₃ and left overnight at +5°C under stirring. The suspension was centrifuged at 27.000 g for 30 minutes at +5°C followed by filtration, if necessary. The precipitate was discarded. The extract was dialysed 3 x 4hrs against 5 mM NH₄HCO₃ followed by 4hrs against purified water. The volume of dialysis water was 20-50 times the volume in the dialysis bag. The dialysed extract is then lyophilised at -80°C and stored in an air tight container at -20°C.

Sera of 21 grass allergic patients and 21 mite (Der p and/or Der f) allergic patients were collected, respectively. Sera of allergy class ≥ 2 were included in the study. Patients had a positive skin prick test and/or presence of IgE antibodies towards the allergens of Phl p and of mites (Der f and Der p), respectively.

Sera containing polyclonal IgG antibodies were produced by immunising rabbits (3-4 months old that never have been immunised before) with the aqueous allergen extracts of Phl p and mites (Der p and Der f), respectively. Sera is collected from coagulated blood and 0.09% NaN₃ is added:

One crossed immunoelectrophoresis is performed for each individual serum by adding the aqueous allergen extract of Phl p or Der p of Der f and respective rabbit antibodies to precipitate the antigens to a CIE plate. The plates were dried in cold air and incubated overnight in small boxes with 300µl patient serum + 7700µl phosphate buffer. The plates were washed 4x10 minutes with 0.9% NaCl and incubated overnight with I¹²⁵- antiIgE (Pharmacia), 300.000 cpm / plate in a total volume of 8 ml. After washing (4 x 10 min with NaCl) the plates were dried and placed in an X-ray cassette, gel-side up and an X-ray film (Retina) on top. The film was exposed at -80°C for 1 day, 3 days and 10 days. The CIE-plates are stained (Coomassie-staining) and each autoradiography was labeled and cut out.

All precipitates (radioactive + non-radioactive precipitates) are detected, scored and numbered. The radioactively stained precipitates are antigens capable of binding to IgE of patient sera and are then allergens, which may be scored by assigning a score of 3 if a precipitate is visible after 1 day (strong), score 2 if visible after 3 days (moderate) and score 1 if visible after 10 days (weak). Precipitates with no radioactive staining of precipitates are antigens not recognised by IgE antibodies of allergic patients and represents unrelated antigens of the present invention.

An Allergogram is drawn using the precipitate numbers from the reference picture and the number of patients reacting to each precipitate.

### Results

### Phl p:

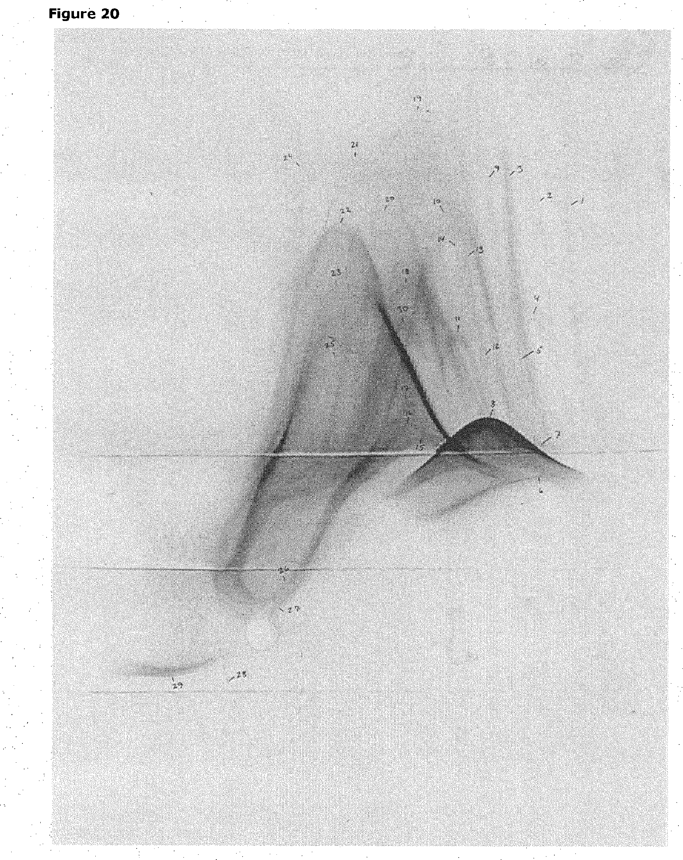

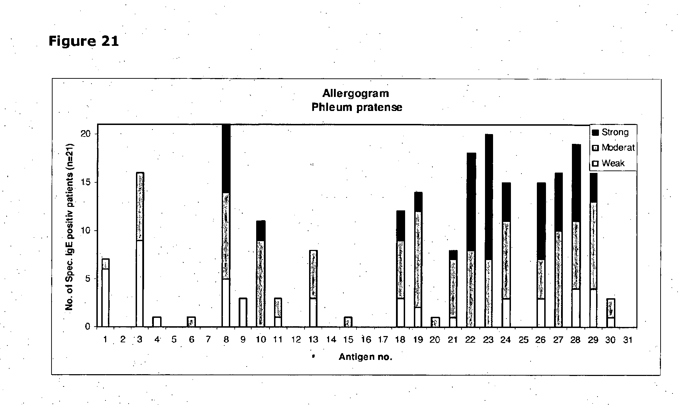

A total of 30 precipitates are visible of which 22 are recognised as allergens and 8 are unrelated antigens of the invention. A CIE diagram (51-990282) is shown in figure 20 and an Allergogram is shown in figure 21. As observed from the Allergogram, the antigen numbered 2, 5, 7, 12, 14, 16, 17 and 31 are non-radioactive precipates and are candidates of an unrelated antigen of the invention.

### Der p:

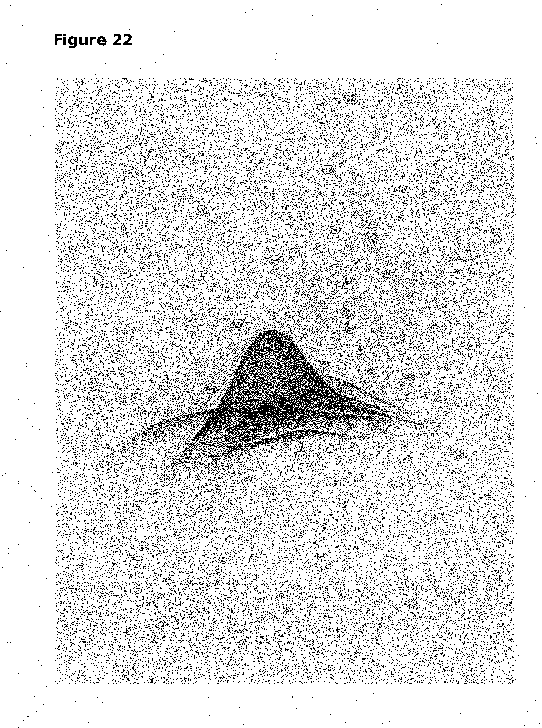

A total of 24 precipitates are visible of which 19 are recognised as allergens and 5 are unrelated antigens of the invention. A CIE diagram is shown in figure 22 and an Allergogram is shown in figure 22. As observed from the Allergogram, the antigen numbered 1, 3, 7, 8, and 9 are non-radioactive precipates and are candidates of an unrelated antigen of the invention.

### Der f:

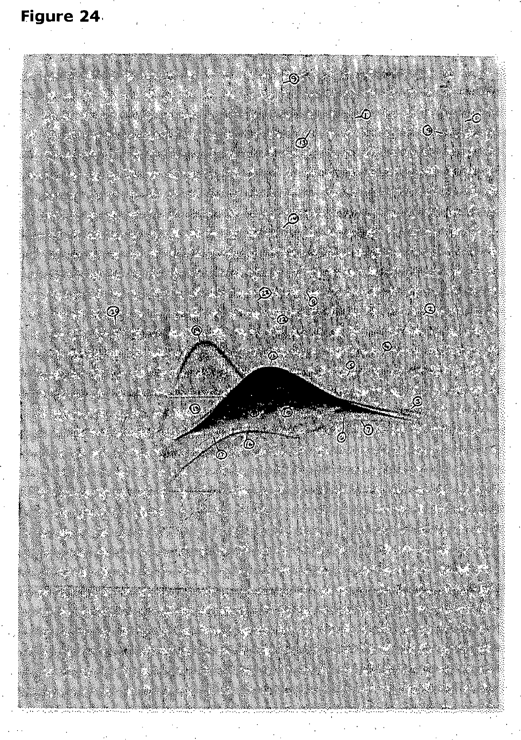

A total of 22 precipitates are visible of which 16 are recognised as allergens and 6 are unrelated antigens of the invention. A CIE diagram is shown in figure 24 and an Allergogram is shown in figure 21. As observed from the Allergogram, the antigen numbered 2, 3, 4, 5, 10 and 17 are non-radioactive precipates and are candidates of an unrelated antigen of the invention.

Thus, unrelated antigens are found in various allergen sources. Further identification of the unrelated antigen may be accomplished by well-known techniques in the art including purifying the precipitate and conducting mass spectrometry analysis.

### Example 8

### Investigating bystander suppression in mice model of Atopic Dermatitis

It will be investigated if SLIT also has a bystander effect in a mouse model of Atopic Dermatitis. For example, allergens obtainable from latex will be used as the "triggering" allergen.

### Methods

Atopic Dermatitis will be induced according to the model developed by Spergel et al (1999) and by Jin H et al (2009). In brief, mice will be sensitized epicutaneously by tape stripping the skin and adminstration of OVA secured by sterile gauze and a bioocclusive dressing. The mice will receive a total of three one week exposures separated by 2 week intervals.

The degree of atopic dermatitis will then be evaluated by obtaining specimens from the patched areas of the skin 24 h after the patch from the third sensitization has been removed.

The specimens will be used for histological analyses to evaluate the thickening of the dermis and epidermis as well as the cellular influx (e.g. eosinophils). Furthermore, the specimens will be stained for cellular markers such as CD3, CD4 and CD8 by immunohistochemistry in order to measure the celluler influx of immune cells. Finally, blood will be collected and cytokines measured using a mesoscale TH1/TH2 ELISA kit.

The effect of SLIT will be investigated in this model by either prophylactic SLIT treatment or by SLIT treatment after the first or second cutaneous administrations of OVA with an antigen non-related to OVA (e.g. Phl p or latex allergen).

### Example 9

### Investigating bystander suppression in mice model of food allergy.

It will be investigated if SLIT also has a bystander effect in a mouse model of food allergy.

### Methods:

Food allergy will be induced according to the model described by Dunkin et al (2011). In brief, mice will be epicutaneously sensitized by administration of 0,1 mg OVA in 50 µL on the abdominal skin of anesthetized mice. The sensitization procedure will be repeated weekly for 6 times. One week after the last sensitization the mice will be orally challenged by increasing oral doses of OVA and symptoms of anaphylaxis as well as body temperature will be recorded every 10 minutes.

In addition to the clinical read-outs mentioned above, blood will be collected from the mice following the oral challenge and specific IgE will be measured in serum. Finally, T-cell reactivity in spleen and local draining lymph nodes will also be measured.

The effect of SLIT will be investigated in this model by either prophylactic SLIT treatment prior to the sensitization or by SLIT treatment after the sensitization procedure, with an antigen non-related to OVA (e.g. Phl p or a peanut allergen).

## Claims

1. A proteinaceous antigen for use in the treatment of a type 1 hypersensitivity immune response of the respiratory tract in an individual in need thereof, wherein
i) the type 1 hypersensitivity immune response is triggered by an allergen upon the individual's exposure to an environmental source material comprising said allergen;
ii) the proteinaceous antigen is unrelated to the allergen(s) triggering the type 1 hypersensitivity immune response in the individual in the sense that it 1) does not bind IgE antibodies derived from said individual in detectable levels in a RAST test
iii) the proteinaceous antigen is administered in a therapeutically effective amount to the sublingual mucosa of said individual,
iv) the proteinaceous antigen is also administered to the respiratory tract within a period at least coinciding partly or entirely with the individual's exposure to said source material, and
v) the proteinaceous antigen is not co-administered together with said allergen.

2. A proteinaceous antigen for use in the treatment of a type 1 hypersensitivity immune response of the gastro-intestinal tract in an individual in need thereof, wherein
i) the immune response is triggered by an allergen upon the individual's exposure to a dietary source material comprising said allergen;
ii) the proteinaceous antigen is unrelated to the allergen(s) triggering the hypersensitivity immune response in the individual in the sense that it 1) does not bind IgE antibodies derived from said individual in detectable levels in a RAST test and/or 2) does not induce a hypersensitivity immune reaction in said individual;
iii) the proteinaceous antigen is administered in a therapeutically effective amount to the sublingual mucosa of said individual,
iv) the proteinaceous antigen is also administered to the gastro-intestinal tract within a period at least coinciding partly or entirely with the individual's exposure to said source material, and
v) the proteinaceous antigen is not co-administered together with said allergen.

3. A proteinaceous antigen for use in the treatment of a type 1 hypersensitivity immune response of the skin in an individual in need thereof, wherein
i) the immune response is triggered by an allergen upon the individual's exposure to an environmental source material comprising said allergen;
ii) the proteinaceous antigen is unrelated to the allergen(s) triggering the hypersensitivity immune response in the individual in the sense that it 1) does not bind IgE antibodies derived from said individual in detectable levels in a RAST test and/or 2) does not induce a hypersensitivity immune reaction in said individual;
iii) the proteinaceous antigen is administered in a therapeutically effective amount to the sublingual mucosa of said individual,
iv) the proteinaceous antigen is also administered to the skin within a period at least coinciding partly or entirely with the individual's exposure to said source material, and
v) the proteinaceous antigen is not co-administered together with said allergen.

4. The proteinaceous antigen for use according to any one of the preceding claims, wherein the proteinaceous antigen has an amino acid sequence having less than 50% identity in relation to the amino acid sequence of the allergen(s).

5. The proteinaceous antigen for use according to any one of the preceding claims, wherein the proteinaceous antigen is a water-soluble antigen.

6. The proteinaceous antigen for use according to any one of the claims 1, 3-5, wherein the environmental source material is selected from the group consisting of mite crops and mite feces of the major taxonomic group Animalia Arthropoda, dander, hair, saliva, stools or other secretes derived from the major taxonomic group Animalia Chordata; spores or particles derived from the major taxonomic group Funghi Ascomycetes, pollen derived from the major taxonomic group Corniferopsida, pollen derived from the major taxonomic group Plantae Magnoliopsida, pollen derived from the major taxonomic group Plantae Liliopsidae; venoms or secretes derived from the major taxonomic group Animalia Arthropoda; gums or products comprising such a gum derived from trees of the major taxonomic group Plantae Magnoliopsidae.

7. The proteinaceous antigen for use according to any one of the claims 1, 3-5, wherein the environmental source material is selected from the group consisting of a crop and/or feces of a mite selected from the group consisting of mites of the genus Acarus, Glycyphagus, Lepidoglyphus, Tyrophagus, Blomia, Dermatophagoides, Euroglyphus, Blatella and Periplaneta; dander, hair, saliva or stools of an animal of a genus selected from canis, felis or Equus; yeast, mould or funghi of a genus selected from the group consisting of Cladosporium, Aspergillus, Penicillium, Alternaria and Candida; pollen of a genus selected from the group consisting of Chamaecyparis, Cryptomeria, Cupressus, Juniperus, Anthoxanthum, Cynodon, Dactylis, Festuca, Holcus, Hordeum, Lolium, Oryza, Paspalum, Phalaris, Phleum, Poa, Secale, Sorghum, Triticum, Zea, Ambrosia, Artemisia, Alnus, Betula, Corylus, Fraxinus, Olea and Platanus; venom of a bee of a genus selected from the group consisting of Apis Bombus, Dolichovespula, Polistes, Polybia, Vespa and Vespula; gum or products comprising such a gum derived from the genus Hevea

8. The proteinaceous antigen for use according to any one of the claims 2, 4, 5, wherein the dietary source material is selected from the group consisting of shrimps or a shrimp-containing food of the major taxonomic group Animalia Arthropoda; lobster or a lobster-containing food of the major taxonomic group Animalia Arthropoda; fruit, legumes, cereals or beans derived from the major taxonomic group Plantae Liliopsidae or a food product comprising such fruits, legumes, cereals and/or beans; fruit, legumes, cereals or beans derived from the major taxonomic group Plantae Magnoliopsidae or a food product comprising such fruits, legumes, cereals and/or beans; nut or a nut-containing food of the from the major taxonomic group Plantae Magnoliopsidae.

9. The proteinaceous antigen for use according to any one of the claims 2, 4, 5, wherein the dietary source material is selected from the group consisting of cow's milk, cow's milk-containing food, chicken egg white, chicken egg white-containing food, fish or fish-containing food.

10. The proteinaceous antigen for use according to any one of the claims 2, 4, 5, wherein the dietary source material is derived from a genus selected from the group consisting of Hordeum, Oryza, Secale, Triticum, Zea, Arachis, Corylis, Juglans, Prunus, Anacardium, Pistacia and Glycine.

11. The proteinaceous antigen for use according to any one of the preceding claims, wherein the proteinaceous antigen is different from an allergen selected from the group consisting of Aca s 13, Gly d 2, Lep d 2, Lep d 5, Lep d 7, Lep d 10, Lep d 13, Tyr p 2, Tyr p 3, Tyr p 10, Tyr p 13, Tyr p 24, Blo t 1, Blo t 2, Blo t 3, Blo t 4, Blo t 5, Blo t 6, Blo t 10, Blo t 11, Blo t 12, Blo t 13, Blo t 19, Blo t 21, Der f 1, Der f 2, Der f 3, Der f 6, Der f 7, Der f 10, Der f 11, Der f 13, Der f 14, Der f 15, Der f 16, Der f 17, Der f 18, Der f 22, Der m 1, Der p 1, Der p 2, Der p 3, Der p 4, Der p 5, Der p 6, Der p 7, Der p 8, Der p 9, Der p 10, Der p 11, Der p 14, Der p 20, Der p 21, Der p 23, Eur m 1, Eur m 2, Eur m 3, Eur m 4, Eur m 14, Bla g 1, Bla g 2, Bla g 4, Bla g 5, Bla g 6, Bla g 7, Bla g 8, Per a 1, Per a 3, Per a 6, Per a 7, Per a 9, Per a 10, Can f 1, Can f 2, Can f 3, Can f 4, Can f 5, Can f 6, Fel d 1, Fel d 2, Fel d 3, Fel d 4, Fel d 5w, Fel d 6w, Fel d 7, Fel d 8, Equ c 1, Equ c 2, Equ c 3, Equ c 4 and Equ c 5, Cla c 9m Cla c 14, Cla h 2, Cla h 5, Cla h 6, Cla h 7, Cla h 8, Cla h 9, Cla h 10, Cla h 12. Asp fl 13, Asp f 1, Asp f 2, Asp f 3, Asp f 4, Asp f 5, Asp f 6, Asp f 7, Asp f 8, Asp f 9, Asp f 10, Asp f 11, Asp f 12, Asp f 13, Asp f 15, Asp f 16, Asp f 17, Asp f 18, Asp f 22, Asp f 23, Asp f 27, Asp f 28, Asp f 29, Asp f 34, Asp n 14, Asp n 18, Asp n 25; Asp o 13, Asp o 21, Pen b 13, Pen b 26, Pen ch 13, Pen ch 18, Pen ch 20, Pen ch 31, Pen ch 33, Pen ch 35, Pen c 3, Pen c 13, Pen c 19, Pen c 22, Pen c 24; Pen c 30, Pen c 32, Pen o 18, Fus c 1, Fus c 2, Alt a 1, Alt a 3, Alt a 4, Alt a 5, Alt a 6, Alt a 7, Alt a 8, Alt a 10, Alt a 12, Alt a 13, Cand a 1, Cand a 3 and Cand b 2, Cha o 1, Cha o 2, Cup a 1, Cup s 1, Cup s 3, Jun a 1, Jun a 2, Jun a 3, Jun o 4, Jun s 1, Jun v 1 and Jun v 3, Ant o 1, Cyn d 1, Cyn d 7, Cyn d 12, Cyn d 15, Cyn d 22w, Cyn d 23, Cyn d 24, Dac g 1, Dac g 2, Dac g 3, Dac g 4, Dac g 5, Fes p 4, Hol l 1, Hol l 5, Hor v 1, Hor v 5, Lol p 1, Lol p 2, Lol p 3, Lol p 4, Lol p 5, Lol p 11, Pas n 1, Pha a 1, Pha a 5, Phl p 1, Phl p 2, Phl p 4, Phl p 5, Phl p 6, Phl p 7, Phl p 11, Phl p 12, Phl p 13, Poa p 1, Poa p 5, Sec c 1, Sec c 5, Sec c 20, Sor h 1, Tri a 15, Tri a 21, Tri a 27, Tri a 28, Tri a 29, Tri a 30, Tri a 31, Tri a 32, Tri a 33, Tri a 34, Tri a 35, Zea m 1 Zea m 12, Amb a 1, Amb a 2, Amb a 3, Amb a 4, Amb a 5, Amb a 6, Amb a 7, Amb a 8, Amb a 9 and Amb a 10; Amb p 5; Amb t 5; Art v 1, Art v 2, Art v 3, Art v 4, Art v 5, Art v 6, Aln g 1, Aln g 4, Bet v 1, Bet v 2, Bet v 3, Bet v 4, Bet v 6, Bet v 7, Cor a 10, Fra e 1, Ole e 1, Ole e 2, Ole e 3, Ole e 4, Ole e 5, Ole e 6, Ole e 7, Ole e 8, Ole e 9, Ole e 10, Ole e 11, Pla a 1, Pla a 2, Pla a 3, Pla or 1, Pla or 2 and Pla or 3, Api c 1, Api d 1; Api m 1, Api m 2, Api m 3, Api m 4, Api m 5, Api m 6, Api m 7, Api m 8, Api m 9, Api m 10, Api m 11, Bom p 1, Bom p 4; Bom t 1, Bom t 4, Dol a 5, Dol m 1, Dol m 2, Dol m 5, Pol a 1, Pol a 2 and Pol a 5, Pol d 1, Pol d 4, Pol d 5, Pol e 1, Pol e 4 and Pol e 5, Pol f 5; Pol g 1, Pol g 5, Poly p 1, Poly s 5; Vesp c 1, Vesp c 5, Vesp ma 2, Vesp ma 5, Vesp m 1, Vesp m 5, Ves g 5, Ves m 1, Ves m 2, Ves m 5; Ves p 5; Ves s 1, Ves s 5; Ves vi 5; Ves v 1, Ves v 2, Ves v 3, Ves v 5, of Hev b 1, Hev b 2, Hev b 3, Hev b 4, Hev b, 5, Hev b 6, Hev b 7, Hev b 8, Hev b 9, Hev b 10, Hev b 11, Hev b 12, Hev b 13 and Hev b 14, Hor v 12, Hor v 15, Hor v 16, Hor v 17, Hor v 21, Ory s 12, Sec c 20, Tri a 12, Tri a 14, Tri a 18, Tri a 19, Tri a 21, Tri a 25, Tri a 26, Tri a 36, Zea m 14, Zea m 25, Ara h 1, Ara h 2, Ara h 3, Ara h 4, Ara h 5, Ara h 6, Ara h 7, Ara h 8, Ara h 9, Ara h 10, Ara h 11, Cor a 1, Cor a 2, Cor a 8, Cor a 9, Cor a 11, Cor a 12, Cor a 13, Cor a 14, Jug n 1, Jug n 2, Jug r 1, Jug r 2, Jug r 3, Jug r 4, Pru du 3, Pru du 4, Pru du 5, Pru du 6, Ana o 1, Ana o 2, Ana o 3, Pis v 1, Pis v 2, Pis v 3, Pis v 4, Pis v 5, Gly m 1, Gly m 2, Gly m 3, Gly m 4, Gly m 5 and Gly m 6.

12. The proteinaceous antigen for use according to any one of claims 1 and 4 to 11, wherein the hypersensitivity immune response is associated with atopic dermatitis, allergic conjunctivitis, allergic rhinitis, allergic asthma, anaphylaxis and/or food allergy.

13. The proteinaceous antigen for use according to any one of claims 2 and 4 to 11, wherein a hypersensitivity immune response of the gastro-intestinal tract is associated to food allergy.

14. The proteinaceous antigen for use according to any one of claims 3 to 11, wherein a hypersensitivity immune response of the skin is associated to atopic dermatitis.

15. The proteinaceous antigen for use according to any one of the preceding claims, which is an immunogenic peptide.

16. The proteinaceous antigen for use according to any one of the preceding claims, which does not initiate an immediate skin reaction in said individual upon conducting skin prick testing with the antigen.

17. The proteinaceous antigen for use according to any one of the preceding claims, which does not induce histamine release in an in vitro basophil/mast cell assay using blood obtained from said individual.

18. The proteinaceous antigen for use according to any one of the preceding claims, which does not share a B-cell epitope with said allergen.

19. The proteinaceous antigen for use according to any one of the preceding claims, which does not bind to IgE antibodies from said individual in detectable levels.

20. The proteinaceous antigen for use according to any one of the preceding claims, wherein symptoms of a type 1 hypersensitivity immune response induced by said allergen are reduced when compared to the symptoms of a type 1 hypersensitivity immune response induced by said allergen in an individual not having been administered the proteinaceous antigen.

## Patentansprüche

1. Proteinartiges Antigen zur Verwendung zur Behandlung einer Hypersensitivitäts-Immunantwort vom Typ 1 der Atmungswege bei einem bedürftigen Individuum, wobei
i) die Hypersensitivitäts-Immunantwort vom Typ 1 durch ein Allergen ausgelöst wird beim Aussetzen des Individuums gegenüber einem Quellmaterial der Umwelt, welches das Antigen umfasst;
ii) das proteinartige Material nicht mit dem/den Antigen(en) verwandt ist, welche die Hypersensitivitäts-Immunantwort vom Typ 1 bei dem Individuum auslösen im Sinne davon, dass es 1) nicht vom Individuum stammende IgE Antikörper in nachweisbaren Mengen in einem RAST-Test bindet und/oder 2) keine Hypersensitivitäts-Immunreaktion bei dem Individuum induziert;
iii) das proteinartige Material in einer therapeutisch wirksamen Menge an die sublinguale Schleimhaut des Individuums verabreicht wird,
iv) das proteinartige Material ebenfalls an die Atmungswege verabreicht wird innerhalb einer Periode, die mindestens teilweise oder vollständig mit dem Aussetzen des Individuums gegenüber dem Quellmaterial übereinstimmt,
v) das proteinartige Antigen nicht gemeinsam mit dem Allergen verabreicht wird.

2. Proteinartiges Antigen zur Verwendung zur Behandlung einer Hypersensitivitäts-Immunantwort vom Typ 1 des Magen-Darm-Trakts bei einem bedürftigen Individuum, wobei
i) die Immunantwort durch ein Allergen ausgelöst wird beim Aussetzen des Individuums gegenüber einem Quellmaterial der Nahrung, welches das Antigen umfasst;
ii) das proteinartige Material nicht mit dem/den Antigen(en) verwandt ist, welche die Hypersensitivitäts-Immunantwort vom Typ 1 bei dem Individuum auslösen im Sinne davon, dass es 1) nicht vom Individuum stammende IgE Antikörper in nachweisbaren Mengen in einem RAST-Test bindet und/oder 2) keine Hypersensitivitäts-Immunreaktion bei dem Individuum induziert;
iii) das proteinartige Material in einer therapeutisch wirksamen Menge an die sublinguale Schleimhaut des Individuums verabreicht wird,
iv) das proteinartige Material ebenfalls an den Magen-Darm-Trakt verabreicht wird innerhalb einer Periode, die mindestens teilweise oder vollständig mit dem Aussetzen des Individuums gegenüber dem Quellmaterial übereinstimmt,
v) das proteinartige Antigen nicht gemeinsam mit dem Allergen verabreicht wird.

3. Proteinartiges Antigen zur Verwendung zur Behandlung einer Hypersensitivitäts-Immunantwort vom Typ 1 der Haut bei einem bedürftigen Individuum, wobei
i) die Immunantwort durch ein Allergen ausgelöst wird beim Aussetzen des Individuums gegenüber einem Quellmaterial der Umwelt, welches das Antigen umfasst;
ii) das proteinartige Material nicht mit dem/den Antigen(en) verwandt ist, welche die Hypersensitivitäts-Immunantwort vom Typ 1 bei dem Individuum auslösen im Sinne davon, dass es 1) nicht vom Individuum stammende IgE Antikörper in nachweisbaren Mengen in einem RAST-Test bindet und/oder 2) keine Hypersensitivitäts-Immunreaktion bei dem Individuum induziert;
iii) das proteinartige Material in einer therapeutisch wirksamen Menge an die sublinguale Schleimhaut des Individuums verabreicht wird,
iv) das proteinartige Material ebenfalls an die Haut verabreicht wird innerhalb einer Periode, die mindestens teilweise oder vollständig mit dem Aussetzen des Individuums gegenüber dem Quellmaterial übereinstimmt,
v) das proteinartige Antigen nicht gemeinsam mit dem Allergen verabreicht wird.

4. Proteinartiges Antigen zur Verwendung nach einem der vorherigen Ansprüche, wobei das proteinartige Antigen eine Aminosäuresequenz aufweist, die weniger als 50 % Identität im Vergleich mit der Aminosäuresequenz des/der Allergens/Allergene aufweist.

5. Proteinartiges Antigen zur Verwendung nach einem der vorherigen Ansprüche, wobei das proteinartige Antigen ein wasserlösliches Antigen ist.

6. Proteinartiges Antigen zur Verwendung nach einem der Ansprüche 1, 3-5, wobei das Quellmaterial der Umwelt ausgewählt wird aus der Gruppe bestehend aus Milbenkulturen und Milbenfäkalien der taxonomischen Hauptgruppe Animalia Arthropoda; Schuppen, Haar, Speichel, Kot oder anderen Sekreten, die von der taxonomischen Hauptgruppe Animalia Chordata stammen; Sporen oder Partikel, die von der taxonomischen Hauptgruppe Funghi Ascomycetes stammen; Pollen, der von der taxonomischen Hauptgruppe Corniferopsida stammt, Pollen, der von der taxonomischen Hauptgruppe Plantae Magnoliopsidae stammt, Pollen, der von der taxonomischen Hauptgruppe Plantae Liliopsidae stammt; Gift oder Sekrete, die von der taxonomischen Hauptgruppe Animalia Arthropoda stammen; Kautschuk oder Produkte, die einen solchen Kautschuk umfassen, der von Bäumen der taxonomischen Hauptgruppe Plantae Magnoliopsidae stammt.

7. Proteinartiges Antigen zur Verwendung nach einem der Ansprüche 1, 3-5, wobei das Quellmaterial der Umwelt ausgewählt wird aus der Gruppe bestehend aus einer Kultur und/oder Fäkalien einer Milbe, die ausgewählt ist aus der Gruppe bestehend aus Milben der Gattung Acarus, Glycyphagus, Lepidoglyphus, Tryophagus, Blomia, Dermatophagoides, Euroglyphus, Blatella und Periplaneta; Schuppen, Haar, Speichel oder Kot eines Tiers einer Gattung ausgewählt aus Canis, Felis oder Equus; Hefe, Schimmelpilz oder Pilze einer Gattung ausgewählt aus der Gruppe bestehend aus Cladosporium, Aspergillus, Penicillium, Alternaria und Candida; Pollen einer Gattung ausgewählt aus der Gruppe bestehend aus Chamaecyparis, Cryptomeria, Cupressus, Juniperus, Anthoxanthum, Cynodon, Dactylis, Festuca, Holcus, Hordeum, Lolium, Oryza, Paspalum, Phalaris, Phleum, Poa, Secale, Sorghum, Triticum, Zea, Ambrosia, Artemisia, Alnus, Betula, Corylus, Fraxinus, Olea und Platanus; Gift einer Biene einer Gattung ausgewählt aus der Gruppe bestehend aus Apis Bombus, Dolichovespula, Polistes, Polybia, Vespa und Vespula; Kautschuk oder Produkte, die einen solchen Kautschuk umfassen, der aus der Gattung Hevea stammt.

8. Proteinartiges Antigen zur Verwendung nach einem der Ansprüche 2, 4, 5, wobei das Quellmaterial der Nahrung ausgewählt wird aus der Gruppe bestehend aus Shrimps oder einer Shrimps-haltigen Nahrung der taxonomischen Hauptgruppe Animalia Arthropoda; Hummer oder einer Hummer-haltigen Nahrung der taxonomischen Hauptgruppe Animalia Arthropoda; Früchte, Hülsenfrüchte, Getreide oder Bohnen, die aus der taxonomischen Hauptgruppe Plantae Liliopsidae stammen oder einem Nahrungsmittelprodukt, das solche Früchte, Hülsenfrüchte, Getreide und/oder Bohnen umfasst; Früchte, Hülsenfrüchte, Getreide oder Bohnen, die aus der taxonomischen Hauptgruppe Plantae Magnoliopsidae stammen oder einem Nahrungsmittelprodukt, das solche Früchte, Hülsenfrüchte, Getreide und/oder Bohnen umfasst; Nuss oder einer nusshaltigen Nahrung der taxonomischen Hauptgruppe Plantae Magnoliopsidae.

9. Proteinartiges Antigen zur Verwendung nach einem der Ansprüche 2, 4, 5, wobei das Quellmaterial der Nahrung ausgewählt wird aus der Gruppe bestehend aus Kuhmilch, Kuhmilch-haltiger Nahrung, Hühnereiweiß, Hühnereiweißhaltiger Nahrung, Fisch oder fischhaltiger Nahrung.

10. Proteinartiges Antigen zur Verwendung nach einem der Ansprüche 2, 4, 5, wobei das Quellmaterial der Nahrung aus einer Gattung stammt, die ausgewählt ist aus der Gruppe bestehend aus Hordeum, Oryza, Secale, Triticum, Zea, Arachis, Corylis, Juglans, Prunus, Anacardium, Pistacia und Glycine.

11. Proteinartiges Antigen zur Verwendung nach einem der vorherigen Ansprüche, wobei sich das proteinartige Antigen von einem Allergen unterscheidet, das ausgewählt ist aus der Gruppe bestehend aus Aca s 13, Gly d 2, Lep d 2, Lep d 5, Lep d 7, Lep d 10, Lep d 13, Tyr p 2, Tyr p 3, Tyr p 10, Tyr p 13, Tyr p 24, Blo t 1, Blo t 2 Blo t 3, Blo t 4, Blo t 5, Blo t 6, Blo t 10, Blo t 11, Blo t 12, Blo t 13, Blo t 19, Blo t 21, Der f 1, Der f 2, Der f 3, Der f 6, Der f 7, Der f 10, Der f 11, Der f 13, Der f 14, Der f 15, Der f 16, Der f 17, Der f 18, Der f 22, Der m 1, Der p 1, Der p 2, Der p 3, Der p 4, Der p 5, Der p 6 Der p 7, Der p 8, Der p 9, Der p 10, Der p 11, Der p 14, Der p 20, Der p 21, Der p 23, Eur m 1, Eur m 2, Eur m 3, Eur m 4, Eur m 14, Bla g 1, Bla g 2, Bla g 4, Bla g 5, Bla g 6, Bla g 7, Bla g 8, Per a 1, Per a 3, Per a 6, Per a 7, Per a 9, Per a 10, Can f 1, Can f 2, Can f 3, Can f 4, Can f 5, Can f 6, Fel d 1, Fel d 2, Fel d 3, Fel d 4, Fel d 5w, Fel d 6w, Fel d 7, Fel d 8, Equ c 1, Equ c 2, Equ c 3, Equ c 4 and Equ c 5, Cla c 9m, Cla c 14, Cla h 2, Cla h 5, Cla h 6, Cla h 7, Ca h 8, Cla h 9, Cla h 10, Cla h 12, Asp fl 13, Asp f 1, Asp f 2, Asp f 3, Asp f 4, Asp f 5, Asp f 6, Asp f 7, Asp f 8, Asp f 9, Asp f 10, Asp f 11, Asp f 12, Asp f 13, Asp f 15, Asp f 16, Asp f 17, Asp f 18, Asp f 22, Asp f 23, Asp f 27, A5p f 28, A5p f 29, Asp f 34, Asp n 14, Asp n 18, Asp n 25, Asp o 13, Asp o 21, Pen b 13, Pen b 26, Pen ch 13, Pen ch 18, Pen ch 20, Pen ch 31, Pen ch 33, Pen ch 35, Pen c 3, Pen c 13, Pen c 19, Pen c 22, Pen c 24, Pen C 30, Pen c 32, Pen o 18, Fus c 1, Fus c 2, Alt a 1, Alt a 3, Alt a 4, Alt a 5, Alt a 6, Alt a 7, Alt a 8, Alt a 10, Alt a 12, Alt a 13, Cand a 1, Cand a 3 and Cand b 2, Cha o 1, Cha o 2, Cup a 1, Cup s 1, Cup s 3, Jun a 1, Jun a 2, Jun a 3, Jun o 4, Jun s 1, Jun v 1 and Jun v 3, Ant o 1, Cyn d 1, Cyn d 7, Cyn d 12, Cyn d 15, Cyn d 22w, Cyn d 23, Cyn d 24, Dac g 1, Dac g 2, Dac g 3, Dac g 4, Dac g 5, Fes p 4, Hol l 1, Hol l 5, Hor v 1, Hor v 5, Lol p 1, Lol p 2, Lol p 3, Lol p 4, Lol p 5, Lol p 11, Pas n 1, Pha a 1, Pha a 5, Phl p 1, Phl p 2, Phl p 4, Phl p 5, Phl p 6, Phl p 7, Phl p 11, Phl p 12, Phl p 13, Poa p 1, Poa p 5, Sec c 1, Sec C 5, Sec c 20, Sor h 1, Tri a 15, Tri a 21, Tri a 27, Tri a 28, Tri a 29, Tri a 30, Tri a 31, Tri a 32, Tri a 33, Tri a 34, Tri a 35, Zea m 1, Zea m 12, Amb a 1, Amb a 2, Amb a 3, Amb a 4, Amb a 5, Amb a 6, Amb a 7, Amb a 8, Amb a 9 and Amb a 10, Amb p 5, Amb t 5, Art v 1, Art v 2, Art v 3, Art v 4, Art v 5, Art v 6, Aln g 1, Aln g 4, Bet v 1, Bet v 2, Bet v 3, Bet v 4, Bet v 6, Bet v 7, Cor a 10, Fra e 1, Ole e 1, Ole e 2, Ole e 3, Ole e 4, Ole e 5, Ole e 6, OIe e 7, Ole e 8, Ole e 9, Ole e 10, Ole e 11, Pla a 1, Pla a 2, Pla a 3, Pla or 1, Pla or 2 and Pla or 3, Api c 1, Api d 1, Api m 1, Api m 2, Api m 3, Api m 4, Api m 5, Api m 6, Api m 7, Api m 8, Api m 9, Api m 10, Api m 11, Bom p 1, Bom p 4, Bom t 1, Bom t 4, Dol a 5, Dol m 1, Dol m 2, Dol m 5, Pol a 1, Pol a 2 and Pol a 5, Pol d 1, Pol d 4, Pol d 5, Pol e 1, Pol e 4 and Pol e 5, PoI f
5, Pol g 1, Pol g 5, Poly p 1, Poly s 5, Vesp c 1, Vesp c 5, Vesp ma 2, Vesp ma 5, Vesp m 1, Vesp m 5, Ves g 5, Ves m 1, Ves m 2, Ves m 5, Ves p 5, Ves s 1, Ves s 5, Ves vi 5, Ves v 1, Ves v 2, Ves v 3, Ves v 5, Hev b 1, Hev b 2, Hev b 3, Hev b 4, Hev b, 5, Hev b 6, Hev b 7, Hev b 8, Hev b 9, Hev b 10, Hev b 11, Hev b 12, Hev b 13 and Hev b 14, Hor v 12, Hor v 15, Hor v 16, Hor v 17, Hor v 21, Ory s 12, Sec c 20, Tri a 12, Tri a 14, Tn a 18, Tri a 19, Tri a 21, Tri a 25, Tri a 26, Tri a 36, Zea m 14, Zea m 25, Ara h 1, Ara h 2, Ara h 3, Ara h 4, Ara h 5, Ara h 6, Ara h 7, Ara h 8, Ara h 9, Ara h 10, Ara h 11, Cor a 1, Cor a 2, Cor a 8, Cor a 9, Cor a 11, Cor a 12, Cor a 13, Cor a 14, Jug n 1, Jug n 2, Jug r 1, Jug r 2, Jug r 3, Jug r 4, Pru du 3, Pru du 4, Pru du 5, Pru du 6, Ana o 1, Ana o 2, Ana o 3, Pis v 1, Pis v 2, Pis v 3, Pis v 4, Pis v 5, Gly m 1, Gly m 2, Gly m 3, Gly m 4, Gly m 5 and Gly m 6.

12. Proteinartiges Antigen zur Verwendung nach einem der Ansprüche 1 und 4-11, wobei die Hypersensitivitäts-Immunantwort in Verbindung steht mit atopischer Dermatitis, allergischer Konjunktivitis, allergischer Rhinitis, allergischem Asthma, Anaphylaxie und/oder Nahrungsmittelallergie.

13. Proteinartiges Antigen zur Verwendung nach einem der Ansprüche 2 und 4-11, wobei eine Hypersensitivitäts-Immunantwort des Magen-Darm-Trakts in Verbindung steht mit Nahrungsmittelallergie.

14. Proteinartiges Antigen zur Verwendung nach einem der Ansprüche 3-11, wobei eine Hypersensitivitäts-Immunantwort der Haut in Verbindung steht mit atopischer Dermatitis.

15. Proteinartiges Antigen zur Verwendung nach einem der vorherigen Ansprüche, das ein immunogenes Peptid ist.

16. Proteinartiges Antigen zur Verwendung nach einem der vorherigen Ansprüche, das keine umittelbare Hautreaktion bei dem Individuum nach Durchführung eines Haut-Pricktests mit dem Antigen auslöst.

17. Proteinartiges Antigen zur Verwendung nach einem der vorherigen Ansprüche, das keine Histaminfreisetzung induziert in einem in vitro Basophilen/Mastzell-Nachweis unter Verwendung von Blut, das von dem Individuum erhalten wird.

18. Proteinartiges Antigen zur Verwendung nach einem der vorherigen Ansprüche, das kein B-Zell Epitop mit dem Allergen gemein hat.

19. Proteinartiges Antigen zur Verwendung nach einem der vorherigen Ansprüche, das nicht in nachweisbaren Mengen IgE Antikörper des Individuums bindet.

20. Proteinartiges Antigen zur Verwendung nach einem der vorherigen Ansprüche, wobei von dem Allergen induzierte Symptome einer Hypersensitivitäts-Immunantwort vom Typ 1 reduziert werden im Vergleich zu den von dem Allergen induzierten Symptomen einer Hypersensitivitäts-Immunantowrt vom Typ 1 bei einem Individuum, dem das proteinartige Antigen nicht verabreicht wurde.

## Revendications

1. Antigène protéique pour son utilisation dans le traitement d'une réponse immunitaire d'hypersensibilité de type 1 du tractus respiratoire chez un individu le nécessitant, dans lequel
i) la réponse immunitaire d'hypersensibilité de type 1 est déclenchée par un allergène sous l'effet de l'exposition de l'individu à un matériau source environnemental comprenant ledit allergène ;
ii) l'antigène protéique n'est pas apparenté à l'allergène (aux allergènes) déclenchant la réponse immunitaire d'hypersensibilité de type 1 chez l'individu dans le sens où 1) il ne se lie pas aux anticorps IgE dérivés dudit individu en des niveaux détectables dans un test RAST, et/ou 2) il n'induit pas de réaction immunitaire d'hypersensibilité chez ledit individu ;
iii) l'antigène protéique est administré en une quantité thérapeutiquement efficace à la muqueuse sublinguale dudit individu,
iv) l'antigène protéique est également administré au tractus respiratoire au cours d'une période coïncidant au moins partiellement ou totalement avec l'exposition de l'individu au dit matériau source, et
v) l'antigène protéique n'est pas administré en même temps que ledit allergène.

2. Antigène protéique pour son utilisation dans le traitement d'une réponse immunitaire d'hypersensibilité de type 1 du tractus gastro-intestinal chez un individu le nécessitant, dans lequel
i) la réponse immunitaire est déclenchée par un allergène sous l'effet de l'exposition de l'individu à un matériau source alimentaire comprenant ledit allergène ;
ii) l'antigène protéique n'est pas apparenté à l'allergène (aux allergènes) déclenchant la réponse immunitaire d'hypersensibilité chez l'individu dans le sens où 1) il ne se lie pas aux anticorps IgE dérivés dudit individu en des niveaux détectables dans un test RAST et/ou 2) il n'induit pas de réaction immunitaire d'hypersensibilité chez ledit individu ;
iii) l'antigène protéique est administré en une quantité thérapeutiquement efficace à la muqueuse sublinguale dudit individu,
iv) l'antigène protéique est également administré au tractus gastro-intestinal au cours d'une période coïncidant au moins partiellement ou totalement avec l'exposition de l'individu au dit matériau source, et
v) l'antigène protéique n'est pas administré en même temps que ledit allergène.

3. Antigène protéique pour son utilisation dans le traitement d'une réponse immunitaire d'hypersensibilité de type 1 de la peau chez un individu le nécessitant, dans lequel
i) la réponse immunitaire est déclenchée par un allergène sous l'effet de l'exposition de l'individu à un matériau source environnemental comprenant ledit allergène ;
ii) l'antigène protéique n'est pas apparenté à l'allergène (aux allergènes) déclenchant la réponse immunitaire d'hypersensibilité chez l'individu dans le sens où 1) il ne se lie pas aux anticorps IgE dérivés dudit individu en des niveaux détectables dans un test RAST et/ou 2) il n'induit pas de réaction immunitaire d'hypersensibilité chez ledit individu ;
iii) l'antigène protéique est administré en une quantité thérapeutiquement efficace à la muqueuse sublinguale dudit individu,
iv) l'antigène protéique est également administré à la peau au cours d'une période coïncidant au moins partiellement ou totalement avec l'exposition dudit individu au dit matériau source, et
v) l'antigène protéique n'est pas administré en même temps que ledit allergène.

4. Antigène protéique pour son utilisation selon l'une quelconque des revendications précédentes, dans lequel l'antigène protéique a une séquence d'acides aminés ayant une identité inférieure à 50 % relativement à la séquence d'acides aminés du ou des allergènes.

5. Antigène protéique pour son utilisation selon l'une quelconque des revendications précédentes, dans lequel l'antigène protéique est un antigène soluble dans l'eau.

6. Antigène protéique pour son utilisation selon l'une quelconque des revendications 1, 3 à 5, dans lequel le matériau source environnemental est sélectionné dans le groupe constitué de colonies d'acariens et d'excréments d'acariens du groupe taxonomique majeur Animalia Arthropoda, de phanères, de cheveux, de salive, de matières fécales ou autres sécrétions dérivés du groupe taxonomique majeur Animalia Chordata ; de spores ou de particules dérivés du groupe taxonomique majeur Funghi Ascomycetes, de pollen dérivé du groupe taxonomique majeur Corniferopsida, de pollen dérivé du groupe taxonomique majeur Plantae Magnoliopsida, de pollen dérivé du groupe taxonomique majeur Plantae Loliopsidae ; de venins ou de sécrétions dérivés du groupe taxonomique majeur Animalia Arthropoda ; de gommes ou de produits comprenant une telle gomme dérivées d'arbres du groupe taxonomique majeur Plantae Magnoliopsidae.

7. Antigène protéique pour son utilisation selon l'une quelconque des revendications 1, 3 à 5, dans lequel le matériau source environnemental est sélectionné dans le groupe constitué d'une colonie et/ou d'excréments d'acariens sélectionnés dans le groupe constitué d'acariens des genres Acarus, Glycyphagus, Lepidoglyphus, Tyrophagus, Blomia, Dermatophagoides, Euroglyphus, Blatella et Periplaneta ; de phanères, de cheveux, de salive ou de matières fécales d'un animal d'un genre sélectionné parmi les genres canis, felis et Equus ; de levure, de moisissure ou de champignons d'un genre sélectionné dans le groupe constitué des genres Cladosporium, Aspergillus, Penicillium, Alternaria et Candida ; de pollen d'un genre sélectionné dans le groupe constitué des genres Chamaecyparis, Cryptomeria, Cupressus, Juniperus, Anthoxanthum, Cynodon, Dactylis, Festuca, Holcus, Hordeum, Lolium, Oryza, Paspalum, Phalaris, Phleum, Poa, Secale, Sorghum, Triticum, Zea, Ambrosia, Artemisia, Alnus, Betula, Corylus, Fraxinus, Olea et Platanus ; de venin d'une abeille d'un genre sélectionné dans le groupe constitué des genres Apis Bombus, Dolichovespula, Polistes, Polybia, Vespa et Vespula ; d'une gomme ou de produits comprenant une telle gomme dérivées du genre Hevea.

8. Antigène protéique pour son utilisation selon l'une quelconque des revendications 2, 4, 5, dans lequel le matériau source alimentaire est sélectionné dans le groupe constitué de crevettes ou d'un aliment contenant des crevettes du groupe taxonomique majeur Animalia Arthropoda ; de homards ou d'un aliment contenant du homard du groupe taxonomique majeur Animalia Arthropoda ; de fruits, de légumineuses, de céréales ou de haricots dérivés du groupe taxonomique majeur Plantae Liliopsidae ou d'un produit alimentaire comprenant ces fruits, légumineuses, céréales et/ou haricots ; de fruits, de légumineuses, de céréales ou de haricots dérivés du groupe taxonomique majeur Plantae Magnoliopsidae ou d'un produit alimentaire comprenant ces fruits, légumineuses, céréales et/ou haricots ; de noix ou d'un aliment contenant des noix du groupe taxonomique majeur Plantae Magnoliopsidae.

9. Antigène protéique pour son utilisation selon l'une quelconque des revendications 2, 4, 5, dans lequel le matériau source alimentaire est sélectionné dans le groupe constitué de lait de vache, d'un aliment contenant du lait de vache, de blanc d'oeuf de poule, d'un aliment contenant du blanc d'oeuf de poule, de poisson ou d'un aliment contenant du poisson.

10. Antigène protéique pour son utilisation selon l'une quelconque des revendications 2, 4, 5, dans lequel le matériau source alimentaire est dérivé d'un genre sélectionné dans le groupe constitué des genres Hordeum, Oryza, Secale, Triticum, Zea, Arachis, Corylis, Juglans, Prunus, Anacardium, Pistacia et Glycine.

11. Antigène protéique pour son utilisation selon l'une quelconque des revendications précédentes, dans lequel l'antigène protéique est différent d'un allergène sélectionné dans le groupe constitué de Aca s 13, Gly d 2, Lep d 2, Lep d 5, Lep d 7, Lep d 10, Lep d 13, Tyr p 2, Tyr p 3, Tyr p 10, Tyr p 13, Tyr p 24, Blo t 1, Blo t 2, Blo t 3, Blo t 4, Blo t 5, Blo t 6, Blo t 10, Blo t 11, Blo t 12, Blo t 13, Blo t 19, Blo t 21, Der f 1, Der f 2, Der f 3, Der f 6, Der f 7, Der f 10, Der f 11, Der f 13, Der f 14, Der f 15, Der f 16, Der f 17, Der f 18, Der f 22, Der m 1, Der p 1, Der p 2, Der p 3, Der p 4, Der p 5, Der p 6, Der p 7, Der p 8, Der p 9, Der p 10, Der p 11, Der p 14, Der p 20, Der p 21, Der p 23, Eur m 1, Eur m 2, Eur m 3, Eur m 4, Eur m 14, Bla g 1, Bla g 2, Bla g 4, Bla g 5, Bla g 6, Bla g 7, Bla g 8, Per a 1, Per a 3, Per a 6, Per a 7, Per a 9 et Per a 10, Can f 1, Can f 2, Can f 3, Can f 4, Can f 5, Can f 6, Fel d 1, Fel d 2, Fel d 3, Fel d 4, Fel d 5w, Fel d 6w, Fel d 7, Fel d 8, Equ c 1, Equ c 2, Equ c 3, Equ c 4 et Equ c 5 ; Cla c 9m, Cla c 14, Cla h 2, Cla h 5, Cla h 6, Cla h 7, Cla h 8, Cla h 9, Cla h 10, Cla h 12. Asp fl 13, Asp f 1, Asp f 2, Asp f 3, Asp f 4, Asp f 5, Asp f 6, Asp f 7, Asp f 8, Asp f 9, Asp f 10, Asp f 11, Asp f 12, Asp f 13, Asp f 15, Asp f 16, Asp f 17, Asp f 18, Asp f 22, Asp f 23, Asp f 27, Asp f 28, Asp f 29, Asp f 34, Asp n 14, Asp n 18, Asp n 25 ; Asp o 13, Asp o 21, Pen b 13, Pen b 26, Pen ch 13, Pen ch 18, Pen ch 20, Pen ch 31, Pen ch 33, Pen ch 35, Pen c 3, Pen c 13, Pen c 19, Pen c 22, Pen c 24 ; Pen c 30, Pen c 32, Pen o 18, Fus c 1, Fus c 2, Alt a 1, Alt a 3, Alt a 4, Alt a 5, Alt a 6, Alt a 7, Alt a 8, Alt a 10, Alt a 12, Alt a 13, Cand a 1, Cand a 3 et Cand b 2, Cha o 1, Cha o 2, Cup a 1, Cup s 1, Cup s 3, Jun a 1, Jun a 2, Jun a 3, Jun o 4, Jun s 1, Jun v 1 et Jun v 3, Ant o 1, Cyn d 1, Cyn d 7, Cyn d 12, Cyn d 15, Cyn d 22w, Cyn d 23, Cyn d 24, Dac g 1, Dac g 2, Dac g 3, Dac g 4, Dac g 5, Fes p 4, Hol 1 1, Hol 1 5, Hor v 1, Hor v 5, Lol p 1, Lol p 2, Lol p 3, Lol p 4, Lol p 5, Lol p 11, Pas n 1, Pha a 1, Pha a 5, Phl p 1, Phl p 2, Phl p 4, Phl p 5, Phl p 6, Phl p 7, Phl p 11, Phl p 12, Phl p 13, Poa p 1, Poa p 5, Sec c 1, Sec c 5, Sec c 20, Sor h 1, Tri a 15, Tri a 21, Tri a 27, Tri a 28, Tri a 29, Tri a 30, Tri a 31, Tri a 32, Tri a 33, Tri a 34, Tri a 35, Zea m 1, Zea m 12, Amb a 1, Amb a 2, Amb a 3, Amb a 4, Amb a 5, Amb a 6, Amb a 7, Amb a 8, Amb a 9 et Amb a 10 ; Amb p 5 ; Amb t 5 ; Art v 1, Art v 2, Art v 3, Art v 4, Art v 5, Art v 6, Aln g 1, Aln g 4, Bet v 1, Bet v 2, Bet v 3, Bet v 4, Bet v 6, Bet v 7, Cor a 10, Fra e 1, Ole e 1, Ole e 2, Ole e 3, Ole e 4, Ole e 5, Ole e 6, Ole e 7, Ole e 8, Ole e 9, Ole e 10, Ole e 11, Pla a 1, Pla a 2, Pla a 3, Pla or 1, Pla or 2 et Pla or 3, Api c 1, Api d 1 ; Api m 1, Api m 2, Api m 3, Api m 4, Api m 5, Api m 6, Api m 7, Api m 8, Api m 9, Api m 10, Api m 11, Bom p 1, Bom p 4 ; Bom t 1, Bom t 4, Dol a 5, Dol m 1, Dol m 2, Dol m 5, Pol a 1, Pol a 2 et Pol a 5, Pol d 1, Pol d 4, Pol d 5, Pol e 1, Pol e 4 et Pol e 5, Pol f 5 ; Pol g 1, Pol g 5, Poly p 1, Poly s 5 ; Vesp c 1, Vesp c 5, Vesp ma 2, Vesp ma 5, Vesp m 1, Vesp m 5, Ves g 5, Ves m 1, Ves m 2, Ves m 5 ; Ves p 5 ; Ves s 1, Ves s 5 ; Ves vi 5 ; Ves v 1, Ves v 2, Ves v 3 et Ves v 5, de Hev b 1, Hev b 2, Hev b 3, Hev b 4, Hev b, 5, Hev b 6, Hev b 7, Hev b 8, Hev b 9, Hev b 10, Hev b 11, Hev b 12, Hev b 13 et Hev b 14 ; Hor v 12, Hor v 15, Hor v 16, Hor v 17, Hor v 21, Ory s 12, Sec c 20, Tri a 12, Tri a 14, Tri a 18, Tri a 19, Tri a 21, Tri a 25, Tri a 26, Tri a 36, Zea m 14, Zea m 25, Ara h 1, Ara h 2, Ara h 3, Ara h 4, Ara h 5, Ara h 6, Ara h 7, Ara h 8, Ara h 9, Ara h 10, Ara h 11, Cor a 1, Cor a 2, Cor a 8, Cor a 9, Cor a 11, Cor a 12, Cor a 13, Cor a 14, Jug n 1, Jug n 2, Jug r 1, Jug r 2, Jug r 3, Jug r 4, Pru du 3, Pru du 4, Pru du 5, Pru du 6, Ana o 1, Ana o 2, Ana o 3, Pis v 1, Pis v 2, Pis v 3, Pis v 4, Pis v 5, Gly m 1, Gly m 2, Gly m 3, Gly m 4, Gly m 5 et Gly m 6.

12. Antigène protéique pour son utilisation selon l'une quelconque des revendications 1 et 4 à 11, dans lequel la réponse immunitaire d'hypersensibilité est associée à une dermite atopique, une conjonctivite allergique, une rhinite allergique, l'asthme allergique, un choc anaphylactique et/ou une allergie alimentaire.

13. Antigène protéique pour son utilisation selon l'une quelconque des revendications 2 et 4 à 11, dans lequel une réponse immunitaire d'hypersensibilité du tractus gastro-intestinal est associée à une allergie alimentaire.

14. Antigène protéique pour son utilisation selon l'une quelconque des revendications 3 à 11, dans lequel une réponse immunitaire d'hypersensibilité de la peau est associée à une dermite atopique.

15. Antigène protéique pour son utilisation selon l'une quelconque des revendications précédentes, qui est un peptide immunogène.

16. Antigène protéique pour son utilisation selon l'une quelconque des revendications précédentes, qui n'initie pas de réaction cutanée immédiate chez ledit individu lors de la réalisation d'un prick test avec l'antigène.

17. Antigène protéique pour son utilisation selon l'une quelconque des revendications précédentes, qui n'induit pas de libération d'histamines dans un dosage des basophiles/mastocytes in vitro en utilisant le sang obtenu dudit individu.

18. Antigène protéique pour son utilisation selon l'une quelconque des revendications précédentes, qui ne partage pas d'épitope des lymphocytes B avec ledit allergène.

19. Antigène protéique pour son utilisation selon l'une quelconque des revendications précédentes, qui ne se lie pas aux anticorps IgE dudit individu en des niveaux détectables.

20. Antigène protéique pour son utilisation selon l'une quelconque des revendications précédentes, dans lequel les symptômes d'une réponse immunitaire d'hypersensibilité de type 1 induite par ledit allergène sont réduits comparé aux symptômes d'une réponse immunitaire d'hypersensibilité de type 1 induite par ledit allergène chez un individu auquel l'antigène protéique n'a pas été administré.
